# EUROPEAN PATENT APPLICATION

(11) **EP 4 799 640 A1**
(43) Date of publication of application: **02.09.2026**
(21) Application number: 25161147.1
(22) Date of filing: 28.02.2025
(51) Int. Cl.: A61K 41/00, A61K 31/197, A61K 49/00, A61P 35/00, A61K 31/366, A61K 45/06

(54) **USE OF DIRECT INTRACRANIAL DRUG DELIVERY SYSTEM IN BRAIN TUMOR TREATMENT**

(71) Applicant: JLP Health GmbH, 1130 Wien (AT)
(72) Inventor: The designation of the inventor has not yet been filed
(74) Representative: Arth, Hans-Lothar

(57) **Abstract**

The present invention relates to composition for use in treating brain tumor in a subject, the composition comprising 5-aminolevulinic acid, wherein, in use, the composition is provided in an direct intracranial drug delivery system to a subject after at least partial surgical resection of the brain tumor, and wherein the direct intracranial drug delivery system releases a therapeutically effective amount of the composition into a resection cavity in a controlled-release manner. Said composition is particularly useful in treating glioblastoma in combination with artemisinin compounds and optionally anti-glioblastoma drugs. Said composition is particularly useful in combination with radiotherapy and/or photodynamic therapy.

## Description

### Field of the invention

The present invention relates to composition for use in treating brain tumor in a subject, the composition comprising 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner. Said composition is particularly useful in treating glioblastoma in combination with artemisinin compounds and optionally anti-glioblastoma drugs. Said composition is particularly useful in combination with radiotherapy and/or photodynamic therapy.

### Background of the invention

5-Aminolevulinic acid (5-ALA) is an early metabolite of the hemoglobin biosynthesis pathway. Exogenous 5-ALA is used as an imaging agent to detect malignant tissue during brain tumor surgery. When provided systemically, 5-ALA is metabolized to the fluorescent metabolite protoporphyrin IX (PpIX), which specifically accumulates in brain tumor tissue. The tumor-specific accumulation has been attributed to i) an impaired blood-brain barrier, ii) upregulation of 5-ALA import carriers, and iii) an altered metabolic configuration of the heme biosynthesis pathway in malignant tissue. The increased production of PpIX in malignant brain tumor cells allows the visualization of malignant tumor tissue by violet-red fluorescence after excitation with blue light at a wavelength of 405 nm.

For the patient, oral administration of 5-ALA several hours prior to surgery facilitates tumor resection and increases the total area of resection linked to overall improved treatment outcome. While a single per oral dose of 20 mg/kg 5-ALA is considered safe, multiple dosing has shown significant adverse events in animals including strong phototoxicity and elevated markers of liver toxicity. It is therefore conceivable that regular systemic treatments with 5-ALA in humans leads to accumulation of heme metabolites, strong phototoxicity and potential hepatotoxicity, particularly when combined with other antineoplastic medications.

Thus, it is the objective of the present invention to provide a composition useful for the treatment of a brain tumor in a subject without severe adverse side effects.

The objective of the present invention is solved by the teaching of the independent claims. Further advantageous features, aspects and details of the invention are evident from the dependent claims, the description, the figures, and the examples of the present application.

### Description of the invention

Surprisingly, the inventors have found that low dose and constant intracranial or intratumoral release of 5-aminolevulinic acid (5-ALA) for several days and up to weeks post-surgery is highly tolerable and shows superior efficacy over other known application routes in the treatment of brain tumor.

The inventors identified a novel dosing regimen of continuous, low-dose intracranial administration of 5-aminolevulinic acid (5-ALA) which provides a safe and effective treatment strategy for brain tumors:
While intermittent intracranial bolus injections of 5-ALA in mice (initially 100 µg/day, reduced to 50 µg/day due to toxicity) showed a positive trend toward tumor growth restriction (see Example 1), this regimen was associated with systemic adverse effects, including weight loss and reduced survival.

Switching to a continuous, low-dose intracranial release of 5-ALA (delivering 50 µg/day) yielded robust antitumor activity without compromising body weight (see Example 2). Notably, this approach was more effective than a systemic high-dose (120 mg/kg) regimen, underscoring the benefits of localized, constant drug delivery.

The inventors were able to further demonstrate the favorable safety profile in mouse studies. In healthy, non-tumor-bearing mice, the continuous intracranial delivery of 5-ALA over one month did not affect body weight, blood cell parameters, or serum toxicity markers (see Example 3).

In addition, testing various 5-ALA doses, either alone or in combination with artesunate (ARS = **1e),** revealed that even an extremely low 5-ALA dose (10 µg/day) significantly reduced tumor growth, while maintaining excellent tolerability (see Example 4).

Although constant intracranial 5-ALA delivery alone significantly reduced tumor growth (see Example 5), its combination with orally administered ARS produced a synergistic antitumor effect. ARS administered as a monotherapy did not limit tumor growth, highlighting the enhanced efficacy achieved through their combination (see Example 6).

Consequently, the experimental data provided herein substantiate that continuous, low-dose intracranial administration of 5-ALA, either alone or in combination with oral administration of ARS, provides a synergistic, potent antineoplastic effect in brain tumor models while minimizing systemic toxicity. This innovative dosing scheme offers a promising, safe, and effective therapeutic approach for the treatment of brain tumors.

Therefore, the present invention is directed to a composition for use in treating brain tumor in a subject, the composition comprising 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner.

The direct intracranial drug delivery system preferably provides the composition comprising 5-ALA in the subject during and after the at least partial surgical brain tumor resection. During the brain tumor resection, the surgeon removes the tumor, as thoroughly as possible. Nevertheless, infiltrative tumor cells may remain. The surgeon assures the hemostasis of surrounding tissues. Following the tumor resection, the tumor bed or tumor cavity or resection cavity is formed, i.e. the area formerly occupied by the cancerous growth. Afterwards, the surgeon proceeds, in case of an implantable direct intracranial drug delivery system, such as a wafer or a patch, to the surgical implantation of the direct intracranial drug delivery system in the tumor bed, formed after the tumor resection.

Thus, the present invention is also directed to a composition for use in treating brain tumor in a subject, the composition comprising 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the direct intracranial drug delivery system is implanted in a tumor bed formed after tumor resection.

The term "at least partial surgical resection" as used herein refers to the surgical procedure in which a portion or entirety of a brain tumor mass is removed from the brain. This includes partial resection, i.e. removal of some, but not all, of the tumor tissue, which may be necessitated by factors such as the tumor's proximity to critical brain structures, patient health considerations, or surgical feasibility, and total resection, i.e. complete removal of the visible tumor mass as determined by intraoperative imaging or post-operative assessment. Thus, the term "at least partial surgical resection" encompasses any surgical intervention aimed at debulking the tumor, reducing its volume, or excising as much of the pathological tissue as is safely achievable, with the intent of minimizing symptoms, improving prognosis, or facilitating adjunct therapies such as radiation or chemotherapy.

Reworded, the present invention is directed to a composition for use in treating brain tumor in a subject, the composition comprising 5-aminolevulinic acid, wherein, in use, the composition is provided by a direct intracranial drug delivery system to a subject after complete surgical resection of the brain tumor, and wherein the direct intracranial drug delivery system releases a therapeutically effective amount of the composition into a resection cavity in a controlled-release manner.

Reworded, the present invention is directed to a composition for use in treating brain tumor in a subject, the composition comprising 5-aminolevulinic acid, wherein, in use, the composition is provided by a direct intracranial drug delivery system to a subject after partial surgical resection of the brain tumor, and wherein the direct intracranial drug delivery system releases a therapeutically effective amount of the composition into remaining brain tumor tissue, i.e. intratumorally and/or a resection cavity in a controlled-release manner.

A "therapeutically effective amount" or "therapeutically effective dosage" of the composition, 5-ALA or a therapeutic agent, is any amount of said composition, 5-ALA or the therapeutic agent that, when used alone or in combination with another therapeutic agent after at least partial surgical resection of the brain tumor, prevents or slows tumor regrowth/recurrence, protects a subject against the onset of the brain tumor or promotes tumor regression evidenced by a decrease in severity of cancer symptoms, particularly tumor size, an increase in frequency and duration of disease symptom-free periods, or a prevention of impairment or disability due to the disease affliction. The ability of the therapeutic agent to promote tumor regression and/or inhibit tumor progression can be evaluated using a variety of methods known to the skilled practitioner, such as in human subjects during clinical trials, in animal model systems predictive of efficacy in humans, or by assaying the activity of the agent in *in vitro* assays. By way of example, an anti-tumor agent promotes tumor regression in a subject. In preferred embodiments, a therapeutically effective amount of 5-ALA promotes tumor regression to the point of eliminating the brain tumor. "Promoting cancer regression" means that administering an effective amount of 5-ALA, alone or in combination with an anti-cancer agent, results in a reduction in tumor growth or size, necrosis of the tumor, a decrease in severity of at least one disease symptom, an increase in frequency and duration of disease symptom-free periods, or a prevention of impairment or disability due to the disease affliction. In addition, the terms "effective" and "effectiveness" with regard to a treatment includes both pharmacological effectiveness and physiological safety. Pharmacological effectiveness refers to the ability of the drug to promote cancer regression in the patient. Physiological safety refers to the level of toxicity, or other adverse physiological effects at the cellular, organ and/or organism level (adverse effects) resulting from administration of the agent.

As used herein, the terms "5-aminolevulinic acid", "delta-aminolevulinic acid", "ALA" and "5-ALA" are used interchangeably and encompass 5-amino-4-oxopentanoic acid. The term "5-aminolevulinic acid" as well as the synonyms thereof encompasses also salts such as the hydrochloride, hydrobromide, and hydroiodide salt and especially the hydrochloride salt thereof.

Suitable salts of "5-aminolevulinic acid" include, but are not limited to, salts with acids such as maleic, fumaric, benzoic, ascorbic, succinic, acetic, formic, oxalic, propionic, tartaric, salicylic, citric, gluconic, lactic, mandelic, cinnamic, oleic, tannic, aspartic, stearic, palmitic, glycolic, glutamic, gluconic, glucaronic, saccharic, isonicotinic, methanesulfonic, ethanesulfonic, p-toluenesulfonic, benzenesulfonic acids, or pamoic (e.g, 1,1'-methylene-bis-(2-hydroxy-3-naphthoate)) acid. Suitable inorganic acids include, but are not limited to, hydrochloric, hydrobromic, hydroiodic, sulfuric, phosphoric, or nitric acid. Preferred is the HCl salt of 5-ALA.

According to the present invention, doses of the composition, of 5-aminolevulinic acid or any other therapeutic agent are expressed as a free base. In other words, even if the considered agent is a pharmaceutically acceptable salt of a small molecule, e.g. the hydrochloride salt of 5-aminolevulinic acid, the doses and daily doses are expressed herein on the bases of the corresponding small molecule free base, e.g. 5-aminolevulinic acid. In the *in vivo* experiments disclosed herein the HCl salt of 5-ALA was used.

The term "shell" refers to the part of an intracranial drug delivery system that completely covers the core. The shell consists of an upper membrane which covers the top of the core, a spacer which horizontally surrounds the core, and a lower membrane which covers the bottom of the core.

The term "wt.%" refers to >>weight %<< and >>percent per weight<<.

The disclosure a component, such as the core, consist of 25 wt.% to 75 wt.% of compound A and 75 wt.% to 25 wt.% of compound B has to be understood in the way that both compounds together have to add up to 100 wt.%.

Consequently, the disclosure
a) a core consisting of:
   5-aminolevulinic acid in a range from 25 wt.% to 75 wt.%,
   the core polymer in a range from 75 wt.% to 25 wt.%, and
   optionally the excipient in the core in a range of 5 wt.% to 15 wt.%, based on the total weight of the core,
has to be understood in the way that in case the excipient is present, e.g. in an amount of 15 wt.%, the remaining 5-ALA and core polymer have to add up to 85 wt.% so that all ingredients together add up to 100 wt.%. Consequently, the core polymer cannot be present in the amount of 75 wt.% anymore, because 5-ALA must be present at least in the amount of 25 wt.% so that the maximum amount of the core polymer is 60 wt.%.

As used herein, the term "treating" or "treatment" encompasses to reversing, alleviating or inhibiting the progress of a disease, disorder or condition, or improvement of one or more symptoms of such disease, disorder or condition, to which such term applies. As used herein, "treating" or "treatment" may also refer to decreasing the probability or incidence of the occurrence of a disease, disorder or condition in a mammal as compared to an untreated control population, or as compared to the same mammal prior to treatment. For example, as used herein, "treating" may refer to preventing a disease, disorder or condition, and may include delaying or preventing the onset of a disease, disorder or condition, or delaying or preventing the symptoms associated with a disease, disorder or condition. As used herein, "treating" may also refer to reducing the severity of a disease, disorder or condition or symptoms associated with such disease, disorder or condition prior to a mammal's affliction with the disease, disorder or condition. Such prevention or reduction of the severity of a disease, disorder or condition prior to affliction relates to the administration of the composition of the present invention, as described herein, to a subject that is not at the time of administration afflicted with the disease, disorder or condition. As used herein the term "treating" may also refer to preventing the recurrence of a disease, disorder or condition or of one or more symptoms associated with such disease, disorder or condition. The terms "treatment" and "therapeutically," as used herein, refer to the act of treating, as "treating" is defined above.

Preferably, a disease, disorder is a cancer selected from brain cancer, in particular glioblastoma.

The term "intracranial" or "intracranially" refers to the injection or administration of 5-ALA and/or an artemisinin compound into the brain tissue. This covers intracerebral delivery, injection or administration into the cerebrospinal fluid (e.g. via intracerebroventricular (ICV) or intrathecal injection), as well as delivery into cavities emerging from brain surgeries.

The term "patient" or "subject," as used herein, refers to a mammalian subject (primates (e.g. humans, cows, sheep, goats, pigs, horses, dogs, cats, rabbits, rats, mice and the like), preferably a human subject (e.g. a man, a woman or a child), that has, is suspected of having, or is or may be susceptible to a condition associated with brain cancer

The term "resection cavity" refers to an anatomical space created in the brain after the surgical removal of a tumor. It comprises the tumor bed, i.e. the area that was occupied by the tumor, and may include surrounding peritumoral tissue that potentially harbors residual microscopic tumor cells. This cavity is clinically significant because it serves as a treatment target (such as direct drug delivery systems) that aim to locally release therapeutic agents to eliminate remaining infiltrative tumor cells and reduce the risk of recurrence.

The term "released into resection cavity" refers to the direct release of the composition comprising 5-ALA into the resection cavity and its immediately adjacent (peritumoral) brain tissue after at least partial surgical resection of the brain tumor and intratumoral, i.e. into the remaining tumor cells, in case, the tumor was not removed completely during surgery. Said release mode is designed to target residual infiltrative tumor cells that remain at the margins of the resection cavity and are a primary cause of recurrence, thereby helping to prevent recurrence while minimizing systemic toxicity.

The term "in a controlled-release manner" refers to the delivery or release of a composition or therapeutic agent such as 5-ALA at a predetermined and regulated rate over a specified period. This mode of release is designed to maintain consistent therapeutic levels of the composition in the target site, minimizing fluctuations in drug concentration that could lead to subtherapeutic effects or toxicity. Controlled release encompasses, but is not necessarily limited to, substantially continuous delivery, and patterned delivery (e.g., intermittent delivery over a period of time that is interrupted by regular or irregular time intervals).

Controlled release as used herein does not refer to delivery of a bolus of the composition, 5-ALA or any therapeutic agent described herein, i.e. administration of a single, concentrated dose of at least 1 mg of 5-ALA or any therapeutic agent over a short period of time of several minutes to a human subject. Controlled release as used herein particularly does not refer to the administration of a single, concentrated dose of at least 1 mg of 5-ALA or any therapeutic agent within seconds to 10 minutes, preferably within 1 to 10 minutes. This includes also a daily bolus application of the composition, 5-ALA or any therapeutic agent described herein. Thus, controlled release as used herein does not refer to the daily administration of a single, concentrated dose of at least 1 mg of 5-ALA or any therapeutic agent within 1 to 5 minutes. The conclusion can be drawn from the mouse data that a bolus administration of 1.00 mg or more of 5-ALA administered within 5 minutes or less should strictly be avoided in order to avoid toxic side effects of the 5-ALA administration.

Reworded, the present invention is directed to a composition for use in treating brain tumor in a subject, the composition comprising 5-aminolevulinic acid, wherein, in use, the composition is provided by a direct intracranial drug delivery system to a subject after at least partial surgical resection of the brain tumor, and wherein the direct intracranial drug delivery system continuously releases a therapeutically effective amount of the composition into a resection cavity at a constant or nearly constant rate over time.

Reworded, the present invention is directed to a composition for use in treating brain tumor in a subject, the composition comprising 5-aminolevulinic acid, wherein, in use, the composition is provided by a direct intracranial drug delivery system to a subject after at least partial surgical resection of the brain tumor, and wherein the direct intracranial drug delivery system continuously releases a therapeutically effective amount of the composition into a resection cavity at a linear or nearly linear rate over time.

"Linear release" and "constant release" are used synonymously herein, since in practice, when a controlled release system is designed to deliver a drug at a fixed rate, the cumulative release is linear over time (linear release). In this sense, "linear" and "constant" release describe the same ideal scenario, where the rate of drug release does not vary with time.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity over 1 to 30 days, preferably 7 to 29 days, more preferably 14 to 28 days.

Specifically, in a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity over 1 to 30 days, preferably 7 to 29 days, more preferably 14 to 28 days after implantation of the direct intracranial drug delivery system in the subject.

Reworded, in a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity over 1 to 30 days, preferably 7 to 29 days, more preferably 14 to 28 days after at least partial surgical resection of the brain tumor.

In an alternative embodiment, the composition is continuously released into the resection cavity over 1 to 50 days or 1 to 40 days or 1 to 30 days, more preferably over 2 to 30 days, more preferably over 3 to 30 days, more preferably over 4 to 30 days, and most preferably over 8 to 30 days.

In a preferred embodiment, the composition is continuously released into the resection cavity over at least 4 days. Thus, the composition for use in treating brain tumor in a subject, comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the composition is continuously released into the resection cavity over at least 4 days.

Preferably, the composition is continuously released into the resection cavity over at least 4 days, more preferably at least 5 days, more preferably at least 6 days, more preferably at least 7 days, more preferably at least 8 days, more preferably at least 9 days, more preferably at least 10 days, more preferably at least 11 days, more preferably at least 12 days, more preferably at least 13 days, more preferably at least 14 days, more preferably at least 15 days, more preferably at least 16 days, more preferably at least 17 days, more preferably at least 18 days, more preferably at least 19 days, more preferably at least 20 days, more preferably at least 21 days, more preferably at least 22 days, more preferably at least 23 days, more preferably at least 24 days, more preferably at least 25 days, more preferably at least 26 days, more preferably at least 27 days, more preferably at least 28 days, more preferably at least 29 days, and more preferably at least 30 days after at least partial surgical resection of the brain tumor.

In an alternative embodiment, the composition is continuously released into the resection cavity between 2 to 40 days or 4 days to 30 days, more preferably between 5 days and 30 days, more preferably between 10 days and 29 days, more preferably between 14 days and 28 days, and most preferably between 16 days and 27 days after at least partial surgical resection of the brain tumor.

In an alternative embodiment, the composition is continuously released into the resection cavity between 3 days to 15 days, more preferably between 4 days and 14 days, more preferably between 5 days and 13 days, more preferably between 6 days and 12 days, and most preferably between 7 days and 11 days after at least partial surgical resection of the brain tumor.

In an alternative embodiment, the composition is continuously released into the resection cavity between 8 days to 20 days, more preferably between 9 days and 19 days, more preferably between 10 days and 18 days, more preferably between 11 days and 17 days, and most preferably between 12 days and 16 days after at least partial surgical resection of the brain tumor.

In an alternative embodiment, the composition is continuously released into the resection cavity between 13 days to 25 days, more preferably between 14 days and 24 days, more preferably between 15 days and 23 days, more preferably between 16 days and 22 days, and most preferably between 17 days and 21 days after at least partial surgical resection of the brain tumor.

In an alternative embodiment, the composition is continuously released into the resection cavity between 18 days to 30 days, more preferably between 19 days and 29 days, more preferably between 20 days and 28 days, more preferably between 21 days and 27 days, and most preferably between 22 days and 26 days after at least partial surgical resection of the brain tumor.

In an alternative embodiment, the composition is continuously released into the resection cavity between 23 days to 35 days, more preferably between 24 days and 34 days, more preferably between 25 days and 33 days, more preferably between 26 days and 32 days, and most preferably between 27 days and 30 days after at least partial surgical resection of the brain tumor.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the 5-aminolevulinic acid in the composition is continuously released at a constant rate of 0.1 mg - 100 mg per day.

Preferably, the 5-aminolevulinic acid in the composition is continuously released at a constant rate of 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, and most preferably at a constant rate of 0.5 mg - 10 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the 5-aminolevulinic acid in the composition is continuously released at a constant rate of 0.5 mg - 50 mg per day, preferably 1.0 mg - 40 mg per day, more preferably 2.0 mg - 30 mg per day, and most preferably 3.0 mg - 20 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the 5-aminolevulinic acid in the composition is continuously released at a constant rate of 0.5 mg - 20 mg per day.

In an alternative embodiment, the 5-aminolevulinic acid in the composition is continuously released at a constant rate of about 0.1 mg per day; more preferably at a constant rate of about 0.2 mg per day; more preferably at a constant rate of about 0.3 mg per day; more preferably at a constant rate of about 0.4 mg per day; more preferably at a constant rate of about 0.5 mg per day; more preferably at a constant rate of about 0.6 mg per day; more preferably at a constant rate of about 0.7 mg per day; more preferably at a constant rate of about 0.8 mg per day; more preferably at a constant rate of about 0.9 mg per day; more preferably at a constant rate of about 1.0 mg per day; more preferably at a constant rate of about 1.1 mg per day; more preferably at a constant rate of about 1.2 mg per day; more preferably at a constant rate of about 1.3 mg per day; more preferably at a constant rate of about 1.4 mg per day; more preferably at a constant rate of about 1.5 mg per day; more preferably at a constant rate of about 1.6 mg per day; more preferably at a constant rate of about 1.7 mg per day; more preferably at a constant rate of about 1.8 mg per day; more preferably at a constant rate of about 1.9 mg per day; more preferably at a constant rate of about 2.0 mg per day; more preferably at a constant rate of about 2.1 mg per day; more preferably at a constant rate of about 2.2 mg per day; more preferably at a constant rate of about 2.3 mg per day; more preferably at a constant rate of about 2.4 mg per day; more preferably at a constant rate of about 2.5 mg per day; more preferably at a constant rate of about 2.6 mg per day; more preferably at a constant rate of about 2.7 mg per day; more preferably at a constant rate of about 2.8 mg per day; more preferably at a constant rate of about 2.9 mg per day; more preferably at a constant rate of about 3.0 mg per day; more preferably at a constant rate of about 3.1 mg per day; more preferably at a constant rate of about 3.2 mg per day; more preferably at a constant rate of about 3.3 mg per day; more preferably at a constant rate of about 3.4 mg per day; more preferably at a constant rate of about 3.5 mg per day; more preferably at a constant rate of about 3.6 mg per day; more preferably at a constant rate of about 3.7 mg per day; more preferably at a constant rate of about 3.8 mg per day; more preferably at a constant rate of about 3.9 mg per day; more preferably at a constant rate of about 4.0 mg per day; more preferably at a constant rate of about 4.1 mg per day; more preferably at a constant rate of about 4.2 mg per day; more preferably at a constant rate of about 4.3 mg per day; more preferably at a constant rate of about 4.4 mg per day; more preferably at a constant rate of about 4.5 mg per day; more preferably at a constant rate of about 4.6 mg per day; more preferably at a constant rate of about 4.7 mg per day; more preferably at a constant rate of about 4.8 mg per day; more preferably at a constant rate of about 4.9 mg per day; more preferably at a constant rate of about 5.0 mg per day; more preferably at a constant rate of about 5.1 mg per day; more preferably at a constant rate of about 5.2 mg per day; more preferably at a constant rate of about 5.3 mg per day; more preferably at a constant rate of about 5.4 mg per day; more preferably at a constant rate of about 5.5 mg per day; more preferably at a constant rate of about 5.6 mg per day; more preferably at a constant rate of about 5.7 mg per day; more preferably at a constant rate of about 5.8 mg per day; more preferably at a constant rate of about 5.9 mg per day; more preferably at a constant rate of about 6.0 mg per day; more preferably at a constant rate of about 6.1 mg per day; more preferably at a constant rate of about 6.2 mg per day; more preferably at a constant rate of about 6.3 mg per day; more preferably at a constant rate of about 6.4 mg per day; more preferably at a constant rate of about 6.5 mg per day; more preferably at a constant rate of about 6.6 mg per day; more preferably at a constant rate of about 6.7 mg per day; more preferably at a constant rate of about 6.8 mg per day; more preferably at a constant rate of about 6.9 mg per day; more preferably at a constant rate of about 7.0 mg per day; more preferably at a constant rate of about 7.1 mg per day; more preferably at a constant rate of about 7.2 mg per day; more preferably at a constant rate of about 7.3 mg per day; more preferably at a constant rate of about 7.4 mg per day; more preferably at a constant rate of about 7.5 mg per day; more preferably at a constant rate of about 7.6 mg per day; more preferably at a constant rate of about 7.7 mg per day; more preferably at a constant rate of about 7.8 mg per day; more preferably at a constant rate of about 7.9 mg per day; more preferably at a constant rate of about 8.0 mg per day; more preferably at a constant rate of about 8.1 mg per day; more preferably at a constant rate of about 8.2 mg per day; more preferably at a constant rate of about 8.3 mg per day; more preferably at a constant rate of about 8.4 mg per day; more preferably at a constant rate of about 8.5 mg per day; more preferably at a constant rate of about 8.6 mg per day; more preferably at a constant rate of about 8.7 mg per day; more preferably at a constant rate of about 8.8 mg per day; more preferably at a constant rate of about 8.9 mg per day; more preferably at a constant rate of about 9.0 mg per day; more preferably at a constant rate of about 9.1 mg per day; more preferably at a constant rate of about 9.2 mg per day; more preferably at a constant rate of about 9.3 mg per day; more preferably at a constant rate of about 9.4 mg per day; more preferably at a constant rate of about 9.5 mg per day; more preferably at a constant rate of about 9.6 mg per day; more preferably at a constant rate of about 9.7 mg per day; more preferably at a constant rate of about 9.8 mg per day; more preferably at a constant rate of about 9.9 mg per day; and most preferably at a constant rate of about 10.0 mg per day.

**In** an alternative embodiment, the 5-aminolevulinic acid in the composition is continuously released at a constant rate of about 100 mg per day; more preferably at a constant rate of about 98 mg per day; more preferably at a constant rate of about 96 mg per day; more preferably at a constant rate of about 94 mg per day; more preferably at a constant rate of about 92 mg per day; more preferably at a constant rate of about 90 mg per day; more preferably at a constant rate of about 88 mg per day; more preferably at a constant rate of about 86 mg per day; more preferably at a constant rate of about 84 mg per day; more preferably at a constant rate of about 82 mg per day; more preferably at a constant rate of about 80 mg per day; more preferably at a constant rate of about 78 mg per day; more preferably at a constant rate of about 76 mg per day; more preferably at a constant rate of about 74 mg per day; more preferably at a constant rate of about 72 mg per day; more preferably at a constant rate of about 70 mg per day; more preferably at a constant rate of about 68 mg per day; more preferably at a constant rate of about 66 mg per day; more preferably at a constant rate of about 64 mg per day; more preferably at a constant rate of about 62 mg per day; more preferably at a constant rate of about 60 mg per day; more preferably at a constant rate of about 58 mg per day; more preferably at a constant rate of about 56 mg per day; more preferably at a constant rate of about 54 mg per day; more preferably at a constant rate of about 52 mg per day; more preferably at a constant rate of about 50 mg per day; more preferably at a constant rate of about 48 mg per day; more preferably at a constant rate of about 46 mg per day; more preferably at a constant rate of about 44 mg per day; more preferably at a constant rate of about 42 mg per day; more preferably at a constant rate of about 40 mg per day; more preferably at a constant rate of about 38 mg per day; more preferably at a constant rate of about 36 mg per day; more preferably at a constant rate of about 34 mg per day; more preferably at a constant rate of about 32 mg per day; more preferably at a constant rate of about 30 mg per day; more preferably at a constant rate of about 28 mg per day; more preferably at a constant rate of about 26 mg per day; more preferably at a constant rate of about 24 mg per day; more preferably at a constant rate of about 22 mg per day; more preferably at a constant rate of about 20 mg per day; more preferably at a constant rate of about 19 mg per day; more preferably at a constant rate of about 18 mg per day; more preferably at a constant rate of about 17 mg per day; more preferably at a constant rate of about 16 mg per day; more preferably at a constant rate of about 15 mg per day; more preferably at a constant rate of about 14 mg per day; more preferably at a constant rate of about 13 mg per day; more preferably at a constant rate of about 12 mg per day; more preferably at a constant rate of about 11 mg per day; more preferably at a constant rate of about 10 mg per day; more preferably at a constant rate of about 9 mg per day; more preferably at a constant rate of about 8 mg per day; more preferably at a constant rate of about 7 mg per day; more preferably at a constant rate of about 6 mg per day; more preferably at a constant rate of about 5 mg per day; more preferably at a constant rate of about 4.5 mg per day; more preferably at a constant rate of about 4 mg per day; more preferably at a constant rate of about 3.5 mg per day; more preferably at a constant rate of about 3.0 mg per day; more preferably at a constant rate of about 2.5 mg per day; more preferably at a constant rate of about 2.0 mg per day; more preferably at a constant rate of about 1.5 mg per day; more preferably at a constant rate of about 1.0 mg per day; more preferably at a constant rate of about 0.9 mg per day; more preferably at a constant rate of about 0.8 mg per day; more preferably at a constant rate of about 0.7 mg per day; more preferably at a constant rate of about 0.6 mg per day; and most preferably at a constant rate of about 0.5 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity over 1 to 50 days or 1 to 40 days or 1 to 30 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

**In** a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 3 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 10 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 20 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 25 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 30 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

### Brain tumor

The inventive composition is particularly useful for treating brain tumor. A **brain tumor** is an abnormal growth of cells within the brain or its surrounding structures, which can be classified as benign (non-cancerous) or malignant (cancerous). Brain tumors may arise from the brain's own tissues, supporting cells, or from metastatic spread from other parts of the body. Brain tumors can be broadly categorized into primary brain tumors (originating in the brain) and secondary/metastatic brain tumors (originating elsewhere and spreading to the brain). Primary brain tumors can be categorized as solid tumors comprising dense, localized masses of abnormal tissue like meningiomas, medulloblastomas, and pituitary adenomas, and gliomas which are tumors arising from glial cells, which support and protect neurons in the central nervous system (CNS).

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the brain tumor is a glioma.

Gliomas are the most common type of primary malignant brain tumors, classified based on the type of glial cell they originate from and their grade of malignancy. Gliomas can be divided into different astrocytomas, including glioblastomas, oligodendrogliomas, ependymomas, and oligoastrocytomas.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the brain tumor is a glioblastoma multiforme.

Reworded, in a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the composition is continuously released into the resection cavity over 1 to 50 days after at least partial surgical resection of the brain tumor, and wherein the brain tumor is a glioblastoma multiforme.

Preferably, the composition is continuously released into the resection cavity over 4 to 50 days, more preferably over 5 to 45 days, more preferably over 6 to 40 days, more preferably over 7 to 35 days, and most preferably over 8 to 30 days.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the composition is continuously released into the resection cavity over at least 4 days after at least partial surgical resection of the brain tumor, and wherein the brain tumor is a glioblastoma multiforme.

Preferably, the composition is continuously released into the resection cavity over at least 5 days, more preferably at least 6 days, more preferably at least 7 days, more preferably at least 8 days, more preferably at least 9 days, more preferably at least 10 days, more preferably at least 11 days, more preferably at least 12 days, more preferably at least 13 days, more preferably at least 14 days, more preferably at least 15 days, more preferably at least 16 days, more preferably at least 17 days, more preferably at least 18 days, more preferably at least 19 days, more preferably at least 20 days, more preferably at least 21 days, more preferably at least 22 days, more preferably at least 23 days, more preferably at least 24 days, more preferably at least 25 days, more preferably at least 26 days, more preferably at least 27 days, more preferably at least 28 days, more preferably at least 29 days, and more preferably at least 30 days after at least partial surgical resection of the brain tumor.

In an alternative embodiment, the composition is continuously released into the resection cavity over 1 to 50 days or 1 to 40 days or 1 to 30 days, more preferably over 2 to 30 days, more preferably over 3 to 30 days, more preferably over 4 to 30 days, more preferably over 8 to 30 days, more preferably between 10 and 29 days, more preferably between 14 and 28 days, more preferably between 16 and 27 days after at least partial surgical resection of the brain tumor.

In an alternative embodiment, the composition is continuously released into the resection cavity between 3 days to 15 days, more preferably between 4 days and 14 days, more preferably between 5 days and 13 days, more preferably between 6 days and 12 days, and most preferably between 7 days and 11 days after at least partial surgical resection of the brain tumor.

In an alternative embodiment, the composition is continuously released into the resection cavity between 8 days to 20 days, more preferably between 9 days and 19 days, more preferably between 10 days and 18 days, more preferably between 11 days and 17 days, and most preferably between 12 days and 16 days after at least partial surgical resection of the brain tumor.

In an alternative embodiment, the composition is continuously released into the resection cavity between 13 days to 25 days, more preferably between 14 days and 24 days, more preferably between 15 days and 23 days, more preferably between 16 days and 22 days, and most preferably between 17 days and 21 days after at least partial surgical resection of the brain tumor.

In an alternative embodiment, the composition is continuously released into the resection cavity between 18 days to 30 days, more preferably between 19 days and 29 days, more preferably between 20 days and 28 days, more preferably between 21 days and 27 days, and most preferably between 22 days and 26 days after at least partial surgical resection of the brain tumor.

In an alternative embodiment, the composition is continuously released into the resection cavity between 23 days to 35 days, more preferably between 24 days and 34 days, more preferably between 25 days and 33 days, more preferably between 26 days and 32 days, and most preferably between 27 days and 30 days after at least partial surgical resection of the brain tumor.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the 5-aminolevulinic acid in the composition is continuously released at a constant rate of 0.1 mg - 100 mg per day, and wherein the brain tumor is a glioblastoma multiforme.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the 5-aminolevulinic acid in the composition is continuously released at a constant rate of 2 mg - 20 mg per day, and wherein the brain tumor is a glioblastoma multiforme.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the 5-aminolevulinic acid in the composition is continuously released at a constant rate of 0.5 mg - 20 mg per day, and wherein the brain tumor is a glioblastoma multiforme.

Preferably, the 5-aminolevulinic acid in the composition is continuously released at a constant rate of 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, and most preferably at a constant rate of 0.5 mg - 10 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity over 1 to 50 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the brain tumor is a glioblastoma multiforme.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 3 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the brain tumor is a glioblastoma multiforme.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 4 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the brain tumor is a glioblastoma multiforme.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 10 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the brain tumor is a glioblastoma multiforme.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 20 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the brain tumor is a glioblastoma multiforme.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 25 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the brain tumor is a glioblastoma multiforme.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for 15 to 30 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the brain tumor is a glioblastoma multiforme.

### Direct intracranial drug delivery system

The term "direct intracranial drug delivery system" or "local intracranial drug delivery system" refers to a drug delivery system designed to administer a composition comprising 5-aminolevulinic acid directly into the intracranial compartment, thereby bypassing the blood-brain barrier, in a controlled-release manner. This system may comprise direct devices, such as wafers or patches, or externally applied devices, such as pumps, or other delivery devices that enable localized, controlled release of a therapeutically active amount of the composition comprising 5-aminolevulinic acid directly into brain tissue, the cranial cavity or the surrounding intracranial environment. This system facilitates localized, controlled, and sustained release of 5-ALA, thereby improving therapeutic efficacy and minimizing systemic side effects. Drug delivery systems in which a therapeutic agent is delivered systemically and which relies on mechanisms (e.g., nanoparticles, prodrugs, or exosomes) to cross the blood-brain barrier are not direct intracranial drug delivery systems. Drug delivery systems which are not capable of administering a composition comprising 5-aminolevulinic acid directly into the intracranial compartment in a controlled-release manner, such as systems for bolus application (e.g. administering a therapeutic agent whereby a single, discrete dose is delivered rapidly to a target site, thereby providing an immediate, high concentration of the agent within a short time frame) are not direct intracranial drug delivery systems according to the present invention.

In a preferred embodiment, the direct intracranial drug delivery system is designed as a pump. Suitable pumps for direct, localized drug delivery to the brain are, for instance, direct peristaltic infusion pumps, electric pumps, osmotic pumps, and microelectromechanical systems-based micropumps. Direct peristaltic infusion pumps are well established clinically and have been shown to safely bypass the blood-brain barrier. These infusion pumps are usually based on peristaltic pump technology, which uses a rotating mechanism to push fluid at a controlled rate, thereby providing continuous, low-rate infusion directly to a target site in the brain. The direct peristaltic infusion pump can be implanted subcutaneously and connected via a catheter to a precise intracranial location. Osmotic pumps use osmotic pressure differences to drive fluid delivery at a constant rate, thereby achieving a nearly constant (zero-order) release rate with stable drug levels and minimized fluctuations. Osmotic pumps are often used in preclinical studies. A small, fully direct osmotic pump, such as those in the ALZET^{®} series, is particularly useful as a direct intracranial drug delivery system for delivering a composition comprising 5-ALA directly into brain tissue, the cranial cavity or the surrounding intracranial environment in a controlled-release manner over a period of multiple days. These pumps are engineered to provide a constant, zero-order infusion rate that is independent of external power sources or physiological variables, making them especially attractive for intracranial applications. When coupled with a catheter and cannula placed at the targeted brain site (via stereotactic methods), said osmotic pump can reliably deliver low volumes of therapeutic agents continuously over the desired duration. This approach minimizes fluctuations in drug concentration and reduces the need for repeated handling or external refilling, which is critical in the sensitive intracranial environment. Thus, in a preferred embodiment, the composition for use in treating brain tumor in a subject, the composition comprises 5-aminolevulinic acid, wherein, in use, the composition is provided by a direct intracranial drug delivery to a subject after at least partial surgical resection of the brain tumor, and wherein the direct intracranial drug delivery system releases a therapeutically effective amount of the composition into a resection cavity in a controlled-release manner, wherein the direct intracranial drug delivery system is a pump system. Preferably, the direct intracranial drug delivery system is an osmotic pump or an electric pump. Preferably, the direct intracranial drug delivery system is an osmotic pump. Preferably, the direct intracranial drug delivery system is an electric pump. More preferably, the direct intracranial drug delivery system is a direct osmotic pump system.

In a preferred embodiment, the direct intracranial drug delivery system is designed as a patch system. A "patch system" as used herein is a direct intracranial drug delivery system designed to deliver a composition comprising 5-ALA to the surface of brain tissue, the resection cavity or the surrounding intracranial environment, and which incorporates a reservoir of the composition comprising 5-ALA along with a rate-controlling membrane or matrix. The patch may optionally adhere to the surface of brain tissue, the resection cavity or the surrounding intracranial environment. The patch may optionally be designed to contact the surface of brain tissue, the resection cavity or the surrounding intracranial environment. The patch is engineered to deliver the composition in a controlled, sustained manner directly to the tissue site, maintaining therapeutic concentrations over an extended period. The patch system may also be designed for conformal contact with irregular tissue surfaces to optimize drug delivery efficiency.

Thus, in a preferred embodiment, the composition for use in treating brain tumor in a subject, the composition comprises 5-aminolevulinic acid, wherein, in use, the composition is provided by a direct intracranial drug delivery to a subject after at least partial surgical resection of the brain tumor, and wherein the direct intracranial drug delivery system releases a therapeutically effective amount of the composition into a resection cavity in a controlled-release manner, wherein the direct intracranial drug delivery system is a patch system.

In a preferred embodiment, the direct intracranial drug delivery system is designed as a wafer system. A "wafer system" is a thin, solid, and often biodegradable polymeric device embedded with a therapeutic agent. Designed for implantation during surgical procedures, the wafer is placed directly onto the brain tissue, the cranial cavity or the surrounding intracranial environment to provide localized and controlled drug release over a predetermined period. Its structure allows for gradual degradation or diffusion, ensuring a sustained delivery profile while minimizing systemic exposure.

Thus, in a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the direct intracranial drug delivery system is a wafer system.

In a particularly preferred embodiment, the direct intracranial drug delivery system is an implantable system for constant release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core.

In a preferred embodiment, the core has the shape of a disc. Thus, the composition for use in treating brain tumor in a subject, the composition comprises 5-aminolevulinic acid, wherein, in use, the composition is provided by a direct intracranial drug delivery system to a subject after at least partial surgical resection of the brain tumor, wherein the direct intracranial drug delivery system releases a therapeutically effective amount of the composition into a resection cavity in a controlled-release manner, and wherein the direct intracranial drug delivery system is an implantable system for constant release of 5-aminolevulinic acid comprising
a) a core disc comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core disc contains 5-aminolevulinic acid in a range from 25 wt.% to 45 wt. %, and the core polymer in a range from 75 wt.% to 55 wt.% based on the total weight of the core disc; or
when the core polymer is poly(lactic-co-glycolic acid) (PLGA), the core disc contains 5-aminolevulinic acid in a range from 65 wt.% to 75 wt.% and the core polymer in a range from 25 wt.% to 35 wt.% based on the total weight of the core disc.

Reworded, the composition for use in treating brain tumor in a subject, the composition comprises 5-aminolevulinic acid, wherein, in use, the composition is provided by a direct intracranial drug delivery system to a subject after at least partial surgical resection of the brain tumor, wherein the direct intracranial drug delivery system releases a therapeutically effective amount of the composition into a resection cavity in a controlled-release manner, and wherein the direct intracranial drug delivery system is delivery system is an implantable system for constant release of 5-aminolevulinic acid comprising
a) a core matrix comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core matrix contains 5-aminolevulinic acid in a range from 25 wt.% to 45 wt. %, and the core polymer in a range from 75 wt.% to 55 wt.% based on the total weight of the core disc; or
when the core polymer is poly(lactic-co-glycolic acid) (PLGA), the core matrix contains 5-aminolevulinic acid in a range from 65 wt.% to 75 wt.% and the core polymer in a range from 25 wt.% to 35 wt.% based on the total weight of the core matrix.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the composition is continuously released into the resection cavity over 1 to 30 days after at least partial surgical resection of the brain tumor, and wherein the direct intracranial drug delivery system is an implantable system for constant release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt. %, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core.

In an alternative embodiment, the composition is continuously released into the resection cavity by the implantable system over 1 to 50 days or 1 to 40 days or 1 to 30 days, more preferably over 2 to 30 days, more preferably over 3 to 30 days, more preferably over 4 to 30 days, and most preferably over 8 to 30 days.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the composition is continuously released into the resection cavity over at least 4 days after at least partial surgical resection of the brain tumor, and wherein the direct intracranial drug delivery system is an implantable system for constant release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core.

Preferably, the composition is continuously released into the resection cavity over at least 4 days, more preferably at least 5 days, more preferably at least 6 days, more preferably at least 7 days, more preferably at least 8 days, more preferably at least 9 days, more preferably at least 10 days, more preferably at least 11 days, more preferably at least 12 days, more preferably at least 13 days, more preferably at least 14 days, more preferably at least 15 days, more preferably at least 16 days, more preferably at least 17 days, more preferably at least 18 days, more preferably at least 19 days, more preferably at least 20 days, more preferably at least 21 days, more preferably at least 22 days, more preferably at least 23 days, more preferably at least 24 days, more preferably at least 25 days, more preferably at least 26 days, more preferably at least 27 days, more preferably at least 28 days, more preferably at least 29 days, and more preferably at least 30 days after at least partial surgical resection of the brain tumor.

In an alternative embodiment, the composition is continuously released into the resection cavity between 4 days to 50 days, more preferably between 15 days and 50 days, more preferably between 15 days and 45 days, more preferably between 20 days and 45 days, and most preferably between 20 days and 35 days after at least partial surgical resection of the brain tumor.

In an alternative embodiment, the composition is continuously released into the resection cavity by the implantable system between 4 days to 30 days, more preferably between 5 days and 30 days, more preferably between 10 days and 30 days, more preferably between 15 days and 30 days, and most preferably between 20 days and 30 days after at least partial surgical resection of the brain tumor

In an alternative embodiment, the composition is continuously released into the resection cavity between 8 days to 20 days, more preferably between 9 days and 19 days, more preferably between 10 days and 18 days, more preferably between 11 days and 17 days, and most preferably between 12 days and 16 days after at least partial surgical resection of the brain tumor.

In an alternative embodiment, the composition is continuously released into the resection cavity between 13 days to 25 days, more preferably between 14 days and 24 days, more preferably between 15 days and 23 days, more preferably between 16 days and 22 days, and most preferably between 17 days and 21 days after at least partial surgical resection of the brain tumor.

In an alternative embodiment, the composition is continuously released into the resection cavity between 18 days to 30 days, more preferably between 19 days and 29 days, more preferably between 20 days and 28 days, more preferably between 21 days and 27 days, and most preferably between 22 days and 26 days after at least partial surgical resection of the brain tumor.

In an alternative embodiment, the composition is continuously released into the resection cavity between 23 days to 35 days, more preferably between 24 days and 34 days, more preferably between 25 days and 33 days, more preferably between 26 days and 32 days, and most preferably between 27 days and 30 days after at least partial surgical resection of the brain tumor.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, wherein the 5-aminolevulinic acid in the composition is continuously released at a constant rate of 0.1 mg - 100 mg per day, and wherein the direct intracranial drug delivery system is an implantable system for constant release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core.

Preferably, the 5-aminolevulinic acid in the composition is continuously released at a constant rate of 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity over 1 to 50 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the direct intracranial drug delivery system
is an implantable system for constant release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 3 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the direct intracranial drug delivery system is an implantable system for constant release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core..

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 10 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the direct intracranial drug delivery system is an implantable system for constant release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 20 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the direct intracranial drug delivery system is an implantable system for constant release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 25 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the direct intracranial drug delivery system is an implantable system for constant release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for 15 to 30 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the direct intracranial drug delivery system is an implantable system for constant release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core.

In a particularly preferred embodiment, the direct intracranial drug delivery system comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core;
wherein the shell further comprises 5-aminolevulinic acid in a range from 1 wt.% to 25 wt.% based on the total weight of the shell.

Preferably, the shell further comprises 5-aminolevulinic acid in a range from 1 wt.% to 20 wt.% based on the total weight of the shell. More preferably, the shell further comprises 5-aminolevulinic acid in a range from 1 wt.% to 15 wt.% based on the total weight of the shell.

In one embodiment, the core and/or the shell of the direct intracranial drug delivery system comprises at least one excipient. In one embodiment, said at least one excipient is an antioxidant selected from ascorbic acid, acetylcysteine, cysteine, thioglycerol, sodium hydrogen sulfite (sodium bisulfite), butylated hydroxyanisole (BHA), butylated hydroxytoluene (BHT), α-tocopherol acetate (vitamin E acetate), methionine, citric acid, ethylenediaminetetraacetic acid (EDTA), tartaric acid, gallic acid and its esters, glutathione, uric acid, carotenoids, and polyphenols. Preferably, the antioxidant is selected from, but not limited to, ascorbic acid, acetylcysteine, cysteine, thioglycerol, sodium hydrogen sulfite, butyl-hydroxyanisole, butyl-hydroxytoluene, α-tocopherol acetate, methionine, and chelators. Further suitable excipients which could be present in the intracranial drug delivery system are organic acids selected from citric acid, fumaric acid, glycolic acid, lactic acid, malic acid, tartaric acid, maleic acid, succinic acid and mixtures of two or more of these organic acids.

In another embodiment, said at least one excipient is an organic acid. **In** a particularly preferred embodiment, the direct intracranial drug delivery system comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core;
and the delivery system further comprising at least one excipient in the core, or in the shell, wherein
the at least one excipient in the core is at least one organic acid having a pKa value in the range of about 1.7 to 4.5, and/or
the at least one excipient in the shell is selected from poly(ethylene glycol) (PEG).

In a particularly preferred embodiment, the direct intracranial drug delivery system comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core;
and the delivery system further comprising at least one excipient in the core, or in the shell, wherein
the at least one excipient in the core is selected from the group consisting of citric acid, fumaric acid, glycolic acid, lactic acid, malic acid, tartaric acid, maleic acid, succinic acid and a mixture thereof; and/or
the at least one excipient in the shell is selected from poly(ethylene glycol) (PEG).

In a particularly preferred embodiment, the direct intracranial drug delivery system comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core;
and the delivery system further comprising at least one excipient in the core, or in the shell, wherein
the at least one excipient in the core is selected from the group consisting of citric acid, fumaric acid, glycolic acid, lactic acid, malic acid, tartaric acid, maleic acid, succinic acid and a mixture thereof; and/or
the at least one excipient in the shell is selected from poly(ethylene glycol) (PEG), and wherein the shell further comprises 5-aminolevulinic acid in a range from 1 wt.% to 25 wt.%, preferably from 1 wt.% to 20 wt.%, more preferably from 1 wt.% to 15 wt.% based on the total weight of the shell.

In a particularly preferred embodiment, the direct intracranial drug delivery system comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core;
and the delivery system further comprising at least one excipient in the core, or in the shell, wherein
the at least one excipient in the core is selected from the group consisting of citric acid, fumaric acid, glycolic acid, lactic acid, malic acid, tartaric acid, maleic acid, succinic acid and a mixture thereof; and/or
the at least one excipient in the shell is selected from poly(ethylene glycol) (PEG); and
the excipient in the core is in a range from 5 wt.% to 15 wt.%, based on the total weight of the core.

In a particularly preferred embodiment, the direct intracranial drug delivery system comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core;
and the delivery system further comprising at least one excipient in the core, or in the shell, wherein
the at least one excipient in the core is selected from the group consisting of citric acid, fumaric acid, glycolic acid, lactic acid, malic acid, tartaric acid, maleic acid, succinic acid and a mixture thereof; and/or
the at least one excipient in the shell is selected from poly(ethylene glycol) (PEG); and
the excipient in the shell is in a range from 35 wt.% to 45 wt.%, based on the total weight of the shell.

In a particularly preferred embodiment, the direct intracranial drug delivery system comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core;
and the delivery system further comprising at least one excipient in the core, or in the shell, wherein
the at least one excipient in the core is selected from the group consisting of citric acid, fumaric acid, glycolic acid, lactic acid, malic acid, tartaric acid, maleic acid, succinic acid and a mixture thereof; and/or
the at least one excipient in the shell is selected from poly(ethylene glycol) (PEG); and
the excipient in the core is in a range from 5 wt.% to 15 wt.%, based on the total weight of the core, and
wherein the excipient in the shell is only contained in the upper membrane and/or the lower membrane of the shell.

In a particularly preferred embodiment, the direct intracranial drug delivery system comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core;
and the delivery system further comprising at least one excipient in the core, or in the shell, wherein
the at least one excipient in the core is selected from the group consisting of citric acid, fumaric acid, glycolic acid, lactic acid, malic acid, tartaric acid, maleic acid, succinic acid and a mixture thereof; and/or
the at least one excipient in the shell is selected from poly(ethylene glycol) (PEG); the excipient in the shell is in a range from 35 wt.% to 45 wt.%, based on the total weight of the shell, and
wherein the excipient in the shell is only contained in the upper membrane and/or the lower membrane of the shell.

In a particularly preferred embodiment, the direct intracranial drug delivery system comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt. %, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core;
and the delivery system further comprising at least one excipient in the core, or in the shell, wherein
the at least one excipient in the core is in a range from 5 wt.% to 15 wt.%, based on the total weight of the core selected from the group consisting of citric acid, fumaric acid, glycolic acid, lactic acid, malic acid, tartaric acid, maleic acid, succinic acid and a mixture thereof; and/or
the at least one excipient in the shell is poly(ethylene glycol) (PEG), and wherein the core is in a range from 40 wt.% to 75 wt.%, preferably from 45 wt.% to 70 wt.% based on the total weight of the implantable system.

**In** a particularly preferred embodiment, the direct intracranial drug delivery system is an implantable system consisting of:
a) a core consisting of:
   5-aminolevulinic acid in a range from 25 wt.% to 75 wt.%,
   the core polymer in a range from 75 wt.% to 25 wt.%, and
   optionally the excipient in the core in a range of 5 wt.% to 15 wt.%, based on the total weight of the core,
   wherein the excipient in the core is citric acid, fumaric acid or a mixture thereof; and
b) a shell consisting of:
   an upper membrane, a spacer, and a lower membrane each comprising
   a shell polymer in a range from 55 wt.% to 100 wt.%, and
   optionally, 5-aminolevulinic acid in a range from 1 wt.% to 15 wt.%, or optionally, the excipient in the shell in a range from 35 wt.% to 45 wt.% based on
   the total weight of the shell,
wherein the excipient in the shell is PEG 400.

In a particularly preferred embodiment, the direct intracranial drug delivery system is an implantable system consisting of:
a) a core consisting of:
   65 wt.% to 75 wt.% of 5-aminolevulinic acid, and 25 wt.% to 35 wt.% of poly (lactic-co-glycolic acid) (PLGA), based on a total weight of the core; and
b) a shell consisting of poly(lactic acid) (PLA);
   wherein in the system poly(lactic acid) (PLA) is in a range from 40 wt.% to 50 wt.%, based on a total weight of the implantable system.

In a particularly preferred embodiment, the direct intracranial drug delivery system is an implantable system consisting of:
a) a core consisting of:
   25 wt.% to 45 wt.% of 5-aminolevulinic acid and 55 wt.% to 75 wt.% of poly(lactic acid) (PLA), based on a total weight of the core; and
b) a shell consisting of poly(lactic acid) (PLA);
   wherein in the system
   poly(lactic acid) (PLA) is in a range from 75 wt.% to 85 wt.%, based on a total weight of the implantable system.

In a particularly preferred embodiment, the direct intracranial drug delivery system is an implantable system consisting of:
a) a core consisting of:
   25 wt.% to 35 wt.% of 5-aminolevulinic acid and 65 wt.% to 75 wt.% of poly(ethylene oxide), based on a total weight of the core; and
b) a shell consisting of poly(lactic acid) (PLA);
   wherein in the system poly(lactic acid) (PLA) is in a range from 45 wt.% to 55 wt.%, based on a total weight of the implantable system.

In a particularly preferred embodiment, the direct intracranial drug delivery system is an implantable system consisting of:
a) a core consisting of:
   65 wt.% to 75 wt.% of 5-aminolevulinic acid and 25 wt.% to 35 wt.% of poly (lactic-co-glycolic acid) (PLGA), based on a total weight of the core; and
b) a shell consisting of 5 wt.% to 15 wt.% of 5-aminolevulinic acid, and 95 wt.% to 85 wt.% of poly(lactic acid) (PLA) based on a total weight of the shell;
   wherein in the system poly(lactic acid) (PLA) is in a range from 35 wt.% to 45 wt.%, based on a total weight of the system.

In a particularly preferred embodiment, the direct intracranial drug delivery system is an implantable system consisting of:
a) a core consisting of:
   25 wt.% to 30 wt.% of 5-aminolevulinic acid, 5 wt.% to 15 wt.% of citric acid or fumaric acid, and 60 wt.% to 70 wt.% of poly(lactic acid) (PLA), based on a total weight of the core; and
b) a shell consisting of poly(lactic acid) (PLA);
   wherein in the system poly(lactic acid) (PLA) is in a range from 75 wt.% to 85 wt.%, based on a total weight of the system.

In a particularly preferred embodiment, the direct intracranial drug delivery system is an implantable system consisting of:
a) a core consisting of:
   65 wt.% to 75 wt.% of 5-aminolevulinic acid, and 25 wt.% to 35 wt.% of poly (lactic-co-glycolic acid) (PLGA) based on a total weight of the core; and
b) a shell consisting of 35 wt.% to 45 wt.% of poly(ethylene glycol) 400, and 55 wt.% to 65 wt.% of poly(lactic acid) (PLA) based on a total weight of the shell
wherein in the system poly(lactic acid) (PLA) is in a range from 25 wt.% to 35 wt.%, based on a total weight of the system.

In a particularly preferred embodiment, the direct intracranial drug delivery system is an implantable system consisting of:
a) a core consisting of:
   25 wt.% to 45 wt.% of 5-aminolevulinic acid, and 75 wt.% to 55 wt.% of poly(lactic acid) (PLA) or poly(ethylene glycol) (PEG) or poly(ethylene oxide) (PEO) based on the total weight of the core;
      or
   65 wt.% to 75 wt.% of 5-aminolevulinic acid, and 25 wt.% to 35 wt.% poly(lactic-co-glycolic acid) (PLGA) based on the total weight of the core;
      and
b) a shell consisting of poly(lactic acid) (PLA);
   wherein in the system poly(lactic acid) (PLA) is in a range from 20 wt.% to 60 wt.%, based on a total weight of the system.

In a particularly preferred embodiment, the direct intracranial drug delivery system is an implantable system in the form of a wafer comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core and the spacer has a thickness (t₄) of at least 0.6 mm.

In a particularly preferred embodiment, the direct intracranial drug delivery system is an implantable system in the form of a wafer comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core,
the spacer has a thickness (t₄) of at least 0.6 mm, and
the core has a thickness (t₁) of 1.5 mm.

In a particularly preferred embodiment, the direct intracranial drug delivery system is an implantable system in the form of a wafer comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly (lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is selected from poly(lactic acid) (PLA);
characterized in that
the shell polymer is in a range from 20 wt.% to 60 wt.% based on the total weight of the implantable system; and
when the core polymer is poly(lactic acid) (PLA), poly(ethylene glycol) (PEG) or poly(ethylene oxide) (PEO), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid) (PLGA), the core contains 5-aminolevulinic acid in a range from 65 wt.% to 75 wt.% and the core polymer in a range from 25 wt.% to 35 wt.% based on the total weight of the core,
the spacer has a thickness (t₄) of at least 0.6 mm, and
the core has a thickness (t₁) of 3.0 mm.

The inventors have further found that oral co-administration of an artemisinin compound together with the controlled-release of the composition described herein by the direct intracranial drug delivery system effectively inhibits tumor growth **(see Examples 2** to **4** and **6**)**.**

Thus, a further aspect of the present invention is directed to a composition for use in treating brain tumor in a subject as described herein in combination with a therapeutically effective amount of an artemisinin compound selected from **1a, 1b, 1c, 1d,** and **1e** or a pharmaceutically acceptable salt thereof 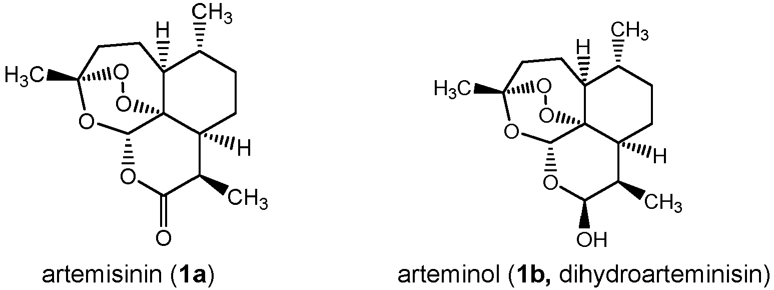 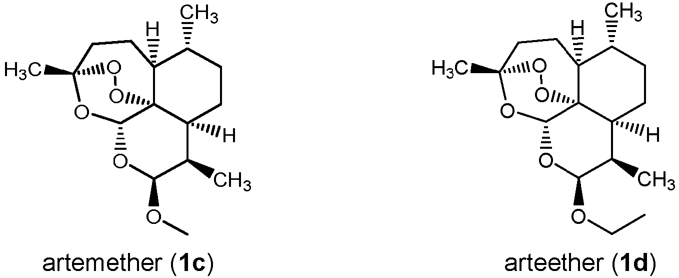 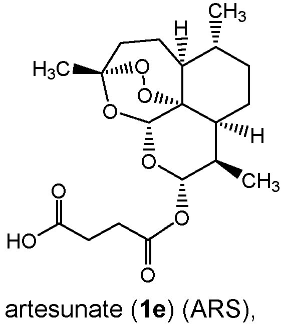 wherein the artemisinin compound is administered systemically or intracranially.

Preferably, the artemisinin compound is administered orally or intravenously and preferably daily to the subject during the release of the therapeutically effective amount of the composition into the resection cavity by the direct intracranial drug delivery system.

As used herein, and unless otherwise specified, the term "pharmaceutically acceptable salt(s) of artemisinin", includes, but is not limited to, salts of acidic or basic moieties of compounds described herein. Basic moieties are capable of forming a wide variety of salts with various inorganic and organic acids. The acids that can be used to prepare pharmaceutically acceptable acid addition salts of such basic compounds are those that form non-toxic acid addition salts, e.g. salts containing pharmacologically acceptable anions. Suitable organic acids include, but are not limited to, maleic, fumaric, benzoic, ascorbic, succinic, acetic, formic, oxalic, propionic, tartaric, salicylic, citric, gluconic, lactic, mandelic, cinnamic, oleic, tannic, aspartic, stearic, palmitic, glycolic, glutamic, gluconic, glucaronic, saccharic, isonicotinic, methanesulfonic, ethanesulfonic, p-toluenesulfonic, benzenesulfonic acids, or pamoic (e.g, 1,1'-methylene-bis-(2-hydroxy-3-naphthoate)) acids. Suitable inorganic acids include, but are not limited to, hydrochloric, hydrobromic, hydroiodic, sulfuric, phosphoric, or nitric acids. Compounds that include an amine moiety can form pharmaceutically acceptable salts with various amino acids, in addition to the acids mentioned above. Chemical moieties that are acidic in nature are capable of forming base salts with various pharmacologically acceptable cations. Examples of such salts are alkali metal or alkaline earth metal salts and, particularly, calcium, magnesium, sodium, lithium, zinc, potassium, or iron salts.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with artesunate (1e)., wherein the direct intracranial drug delivery system releases a therapeutically effective amount of the composition into a resection cavity in a controlled-release manner, and wherein the artesunate (**1e**) is daily administered orally or intravenously to the subject during the release of the therapeutically effective amount of the composition into the resection cavity by the direct intracranial drug delivery system.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound selected from **1a, 1b, 1c, 1d,** and **1e** or a pharmaceutically acceptable salt thereof, wherein the direct intracranial drug delivery system releases a therapeutically effective amount of the composition into a resection cavity in a controlled-release manner, wherein the artemisinin compound is administered daily administered orally or intravenously to the subject during the release of the therapeutically effective amount of the composition into the resection cavity by the direct intracranial drug delivery system, and wherein the daily dose of the artemisinin compound is in a range of 0.5 mg artemisinin compound per kg body weight to 10 mg artemisinin compound per kg body weight.

Preferably, the daily dose of the artemisinin compound is between about 0.1 mg and 100 mg artemisinin compound per kg body weight, more preferably about 0.2 mg and 80 mg artemisinin compound per kg body weight, more preferably about 0.3 mg and 50 mg artemisinin compound per kg body weight, more preferably about 0.4 mg and 25 mg artemisinin compound per kg body weight, more preferably about 0.5 mg and 10 mg artemisinin compound per kg body weight, more preferably about 1 mg and 8 mg artemisinin compound per kg body weight, more preferably about 2 mg and 6 mg artemisinin compound per kg body weight, and most preferably about 3 mg artemisinin compound per kg body weight.

Preferably, the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound selected from **1a, 1b, 1c, 1d,** and **1e** or a pharmaceutically acceptable salt thereof, wherein the direct intracranial drug delivery system releases a therapeutically effective amount of the composition into a resection cavity in a controlled-release manner, wherein the artemisinin compound is administered daily administered orally or intravenously to the subject during the release of the therapeutically effective amount of the composition into the resection cavity by the direct intracranial drug delivery system, and wherein the total daily dose of the artemisinin compound is in a range of 100 mg to 300 mg, preferably 150 mg to 250 mg, more preferably 180 mg to 220 mg, and most preferably about 200 mg.

Preferably, the total daily dose of the artemisinin compound is 100 mg, more preferably 105 mg, more preferably 110 mg, more preferably 115 mg, more preferably 120 mg, more preferably 125 mg, more preferably 130 mg, more preferably 135 mg, more preferably 140 mg, more preferably 145 mg, more preferably 150 mg, more preferably 155 mg, more preferably 160 mg, more preferably 165 mg, more preferably 170 mg, more preferably 175 mg, more preferably 180 mg, more preferably 185 mg, more preferably 190 mg, more preferably 195 mg, and most preferably 200 mg.

Preferably, the total daily dose of the artemisinin compound is 300 mg, more preferably 295 mg, more preferably 290 mg, more preferably 285 mg, more preferably 280 mg, more preferably 275 mg, more preferably 270 mg, more preferably 265 mg, more preferably 260 mg, more preferably 255 mg, more preferably 250 mg, more preferably 245 mg, more preferably 240 mg, more preferably 235 mg, more preferably 230 mg, more preferably 225 mg, more preferably 220 mg, more preferably 215 mg, more preferably 210 mg, more preferably 205 mg, and most preferably 200 mg.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound selected from **1a, 1b, 1c, 1d,** and **1e** or a pharmaceutically acceptable salt thereof, wherein the direct intracranial drug delivery system releases a therapeutically effective amount of the composition into a resection cavity in a controlled-release manner, wherein the artemisinin compound is administered daily administered orally or intravenously to the subject during the release of the therapeutically effective amount of the composition into the resection cavity by the direct intracranial drug delivery system, and wherein a dose of the artemisinin compound of 100 mg is administered twice daily to the subject (e.g. in the morning 100 mg artemisinin compound and in the evening 100 mg artemisinin compound).

In one embodiment, the composition is continuously released into the resection cavity over 1 to 50 days or 1 to 40 days or 1 to 30 days, more preferably over 2 to 30 days, more preferably over 3 to 30 days, more preferably over 4 to 30 days, and most preferably over 8 to 30 days together with a daily oral administration of the artemisinin compound.

In one embodiment, the composition is continuously released into the resection cavity over at least 5 days, more preferably at least 6 days, more preferably at least 7 days, more preferably at least 8 days, more preferably at least 9 days, more preferably at least 10 days, more preferably at least 11 days, more preferably at least 12 days, more preferably at least 13 days, more preferably at least 14 days, more preferably at least 15 days, more preferably at least 16 days, more preferably at least 17 days, more preferably at least 18 days, more preferably at least 19 days, more preferably at least 20 days, more preferably at least 21 days, more preferably at least 22 days, more preferably at least 23 days, more preferably at least 24 days, more preferably at least 25 days, more preferably at least 26 days, more preferably at least 27 days, more preferably at least 28 days, more preferably at least 29 days, and more preferably at least 30 days after at least partial surgical resection of the brain tumor together with a daily oral administration of the artemisinin compound.

In one embodiment, the composition is continuously released into the resection cavity between 4 days to 30 days, more preferably between 5 days and 30 days, more preferably between 10 days and 29 days, more preferably between 14 days and 28 days, and most preferably between 16 days and 27 days after at least partial surgical resection of the brain tumor together with a daily oral administration of the artemisinin compound.

In another embodiment, the composition is continuously released into the resection cavity together with a daily oral administration of the artemisinin compound between 3 days to 15 days, more preferably between 4 days and 14 days, more preferably between 5 days and 13 days, more preferably between 6 days and 12 days, and most preferably between 7 days and 11 days after at least partial surgical resection of the brain tumor.

In another embodiment, the composition is continuously released into the resection cavity together with a daily oral administration of the artemisinin compound between 8 days to 20 days, more preferably between 9 days and 19 days, more preferably between 10 days and 18 days, more preferably between 11 days and 17 days, and most preferably between 12 days and 16 days after at least partial surgical resection of the brain tumor.

In another embodiment, the composition is continuously released into the resection cavity together with a daily oral administration of the artemisinin compound between 13 days to 25 days, more preferably between 14 days and 24 days, more preferably between 15 days and 23 days, more preferably between 16 days and 22 days, and most preferably between 17 days and 21 days after at least partial surgical resection of the brain tumor.

In another embodiment, the composition is continuously released into the resection cavity together with a daily oral administration of the artemisinin compound between 18 days to 30 days, more preferably between 19 days and 29 days, more preferably between 20 days and 28 days, more preferably between 21 days and 27 days, and most preferably between 22 days and 26 days after at least partial surgical resection of the brain tumor.

In another embodiment, the composition is continuously released into the resection cavity together with a daily oral administration of the artemisinin compound between 23 days to 35 days, more preferably between 24 days and 34 days, more preferably between 25 days and 33 days, more preferably between 26 days and 32 days, and most preferably between 27 days and 30 days after at least partial surgical resection of the brain tumor.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously for at least 3 days to the subject during the release of the therapeutically effective amount of the composition into the resection cavity by the direct intracranial drug delivery system, and wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously for at least 10 days to the subject during the release of the therapeutically effective amount of the composition into the resection cavity by the direct intracranial drug delivery system, and wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously for at least 15 days to the subject during the release of the therapeutically effective amount of the composition into the resection cavity by the direct intracranial drug delivery system, and wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously for at least 20 days to the subject during the release of the therapeutically effective amount of the composition into the resection cavity by the direct intracranial drug delivery system, and wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously for at least 25 days to the subject during the release of the therapeutically effective amount of the composition into the resection cavity by the direct intracranial drug delivery system, and wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously for at least 30 days to the subject during the release of the therapeutically effective amount of the composition into the resection cavity by the direct intracranial drug delivery system, and wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 3 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 10 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 15 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 20 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 25 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 30 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 3 days, and wherein the mass ratio of administered 5-ALA to the artemisinin compound **1e** is between 1:1 and 1:3000, preferably between 1:2 and 1:2000, more preferably between 1:3 and 1:1000, more preferably between 1:5 and 1:500, more preferably between 1:6 and 1:400, more preferably between 1:7 and 1:300, more preferably between 1:8 and 1:200, more .preferably between 1:9 and 1:150, and most preferably between 1:10 and 1:100.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 10 days, and wherein the mass ratio of administered 5-ALA to the artemisinin compound **1e** is between 1:1 and 1:3000, preferably between 1:2 and 1:2000, more preferably between 1:3 and 1:1000, more preferably between 1:5 and 1:500, more preferably between 1:6 and 1:400, more preferably between 1:7 and 1:300, more preferably between 1:8 and 1:200, more .preferably between 1:9 and 1:150, and most preferably between 1:10 and 1:100.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 15 days, and wherein the mass ratio of administered 5-ALA to the artemisinin compound **1e** is between 1:1 and 1:3000, preferably between 1:2 and 1:2000, more preferably between 1:3 and 1:1000, more preferably between 1:5 and 1:500, more preferably between 1:6 and 1:400, more preferably between 1:7 and 1:300, more preferably between 1:8 and 1:200, more .preferably between 1:9 and 1:150, and most preferably between 1:10 and 1:100.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 20 days, and wherein the mass ratio of administered 5-ALA to the artemisinin compound **1e** is between 1:1 and 1:3000, preferably between 1:2 and 1:2000, more preferably between 1:3 and 1:1000, more preferably between 1:5 and 1:500, more preferably between 1:6 and 1:400, more preferably between 1:7 and 1:300, more preferably between 1:8 and 1:200, more .preferably between 1:9 and 1:150, and most preferably between 1:10 and 1:100.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 25 days, and wherein the mass ratio of administered 5-ALA to the artemisinin compound **1e** is between 1:1 and 1:3000, preferably between 1:2 and 1:2000, more preferably between 1:3 and 1:1000, more preferably between 1:5 and 1:500, more preferably between 1:6 and 1:400, more preferably between 1:7 and 1:300, more preferably between 1:8 and 1:200, more .preferably between 1:9 and 1:150, and most preferably between 1:10 and 1:100.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 30 days, and wherein the mass ratio of administered 5-ALA to the artemisinin compound **1e** is between 1:1 and 1:3000, preferably between 1:2 and 1:2000, more preferably between 1:3 and 1:1000, more preferably between 1:5 and 1:500, more preferably between 1:6 and 1:400, more preferably between 1:7 and 1:300, more preferably between 1:8 and 1:200, more .preferably between 1:9 and 1:150, and most preferably between 1:10 and 1:100.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 7 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid in the composition is continuously released at a constant rate of about 5 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 7 days, wherein the daily dose of the artemisinin compound is about 3 mg artemisinin compound per kg body weight, and wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 7 days, wherein the daily dose of the artemisinin compound is about 3 mg artemisinin compound per kg body weight, and wherein the 5-aminolevulinic acid in the composition is continuously released at a constant rate of about 1 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 7 days, wherein the daily dose of the artemisinin compound is about 3 mg artemisinin compound per kg body weight, and wherein the 5-aminolevulinic acid in the composition is continuously released at a constant rate of about 5 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 7 days, wherein the daily dose of the artemisinin compound is about 3 mg artemisinin compound per kg body weight, and wherein the 5-aminolevulinic acid in the composition is continuously released at a constant rate of about 10 mg per day.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a controlled-release manner, wherein the artemisinin compound is daily administered orally or intravenously and simultaneously with the controlled release of the composition into the resection cavity for at least 7 days, wherein the daily dose of the artemisinin compound is about 3 mg artemisinin compound per kg body weight, and wherein the 5-aminolevulinic acid in the composition is continuously released at a constant rate of about 15 mg per day.

The artemisinin compound will typically be administered together with a suitable acceptable carrier selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices and could also be administered together with a carrier promoting crossing the blood brain barrier such as virus like particles, neurotropic viruses, exosomes, and nanoparticles. For example, for oral administration in the form of tablets or capsules, the artemisinin compound may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the artemisinin compound or the respective pharmaceutically active salt as active ingredient.

Suitable binders include starch, gelatin, natural sugars, corn sweeteners, natural and synthetic gums such as acacia, sodium alginate, carboxymethylcellulose, poly(ethylene glycol) and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the artemisinin compound may be formulated in sustained release formulation to provide the rate controlled release of the artemisinin compound to optimise the therapeutic effect(s), e.g. anti-cancer activity or activity against cancer metastases and the like. Suitable dosage forms for sustained release include tablets having layers of varying disintegration rates or controlled release, polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn, rice, and potato, and celluloses such as microcrystalline cellulose. The amount of diluent in the composition can range from about 5 to about 95 % by weight of the total composition, preferably from about 25 to about 75 weight%, and more preferably from about 30 to about 60 weight%.

The term disintegrants refers to materials added to the composition to support break apart (disintegrate) and release the artemisinin compound. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight% of the composition, more preferably from about 5 to about 10 weight%.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat, corn, rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight% of the composition, preferably from about 3 to about 10 weight %, and more preferably from about 3 to about 6 weight%.

Lubricants refer to a class of substances which are added to the dosage form to enable the tablet granules etc. after being compressed to release from the mould by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, poly(ethylene glycol)s and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight% of the final composition, preferably from about 0.5 to about 2 weight%.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight% of the composition, preferably from about 0.1 to 1 weight %.

### Chemotherapeutic agents

The composition for use in treating brain tumor described herein may be further combined with oral administration of anti-glioblastoma drug to effectively inhibit postoperative tumor growth.

Therefore, another aspect of the present invention is directed to a composition for use in treating brain tumor in a subject, the composition comprises 5-aminolevulinic acid, wherein, in use, the composition is provided by a direct intracranial drug delivery system to a subject after at least partial surgical resection of the brain tumor in combination with a therapeutically effective amount of at least one anti-glioblastoma drug, wherein the direct intracranial drug delivery system releases a therapeutically effective amount of the composition into a resection cavity in a controlled-release manner, and wherein the therapeutically effective amount of at least one anti-glioblastoma drug is administered systemically to the subject during the release of the therapeutically effective amount of the composition into the resection cavity by the direct intracranial drug delivery system.

In a preferred embodiment, the composition for use in treating brain tumor in a subject, the composition comprises 5-aminolevulinic acid, wherein, in use, the composition is provided in an direct intracranial drug delivery system to a subject after at least partial surgical resection of the brain tumor in combination with a therapeutically effective amount of at least one anti-glioblastoma drug, and wherein the direct intracranial drug delivery system releases a therapeutically effective amount of the composition into a resection cavity in a controlled-release manner, and wherein the at least one anti-glioblastoma drug selected from the group consisting of temozolomide, dexamethasone, lomustine, methotrexate, everolimus, carmustine, cyclophosphamide, cisplatin, carboplatin, 5-fluorouracil, triptolide, homoharringtonin, dactinomycin, doxorubicin, epirubicin, idarubicin, ribavirin, topotecan, flubendazole, itraconazole, vindesine sulfate, cerivastatin, vincristine, vinorebine, nisoldipine, deoxyadenosine, chloro-2'-deoxyadenosine, 5-nonyloxytryptamine, 2(1H)-pyrimidinone, pitavastatin, sertraline, irinotecan, clofazimine, tovorafenib, and docetaxel, and wherein the therapeutically effective amount of at least one anti-glioblastoma drug is administered systemically to the subject during the release of the therapeutically effective amount of the composition into the resection cavity by the direct intracranial drug delivery system.

In a preferred embodiment, the composition for use in treating brain tumor in a subject, the composition comprises 5-aminolevulinic acid, wherein, in use, the composition is provided in an direct intracranial drug delivery system to a subject after at least partial surgical resection of the brain tumor in combination with a therapeutically effective amount of at least one anti-glioblastoma drug, and wherein the direct intracranial drug delivery system releases a therapeutically effective amount of the composition into a resection cavity in a controlled-release manner, and wherein the at least one anti-glioblastoma drug selected from the group consisting of temozolomide, lomustine, cisplatin, and 5 fluorouracil., and wherein the therapeutically effective amount of at least one anti-glioblastoma drug is administered systemically to the subject during the release of the therapeutically effective amount of the composition into the resection cavity by the direct intracranial drug delivery system.

In a preferred embodiment, the composition for use in treating brain tumor in a subject, the composition comprises 5-aminolevulinic acid, wherein, in use, the composition is provided in an direct intracranial drug delivery system to a subject after at least partial surgical resection of the brain tumor in combination with a therapeutically effective amount of temozolomide, wherein the direct intracranial drug delivery system releases a therapeutically effective amount of the composition into a resection cavity in a controlled-release manner, and wherein the therapeutically effective amount of temozolomide is administered systemically to the subject during the release of the therapeutically effective amount of the composition into the resection cavity by the direct intracranial drug delivery system.

In a preferred embodiment, the composition for use in treating brain tumor in a subject, the composition comprises 5-aminolevulinic acid, wherein, in use, the composition is provided in an direct intracranial drug delivery system to a subject after at least partial surgical resection of the brain tumor in combination with a therapeutically effective amount of at least one anti-glioblastoma drug, and wherein the direct intracranial drug delivery system releases a therapeutically effective amount of the composition into a resection cavity in a controlled-release manner, wherein the therapeutically effective amount of at least one anti-glioblastoma drug is administered systemically to the subject during the release of the therapeutically effective amount of the composition into the resection cavity by the direct intracranial drug delivery system in a range of 0.01 to 100 mg/kg per body weight per day.

Preferred, the dose of the anti-glioblastoma drug is administered orally daily is at least 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, 1.00, 1.05, 1.10, 1.15, 1.20, 1.25, 1.30, 1.35, 1.40, 1.45, 1.50, 2.00, 2.50, 3.00, 3.50, 4.00, 4.50, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5 or 15.0 mg/kg anti-glioblastoma drug per body weight per day.

In a preferred embodiment the composition for use in treating brain tumor in a subject comprises 5-aminolevulinic acid, wherein, in use, the composition is provided in an direct intracranial drug delivery system to a subject after at least partial surgical resection of the brain tumor in combination with a therapeutically effective amount of an artemisinin compound selected from **1a, 1b, 1c, 1d,** and **1e** or a pharmaceutically acceptable salt thereof, and at least one anti-glioblastoma drug, wherein the direct intracranial drug delivery system releases a therapeutically effective amount of the composition into a resection cavity in a controlled-release manner, wherein the artemisinin compound is daily administered orally or intravenously to the subject during the release of the therapeutically effective amount of the composition into the resection cavity by the direct intracranial drug delivery system, and wherein the therapeutically effective amount of at least one anti-glioblastoma drug is administered systemically to the subject during the release of the therapeutically effective amount of the composition into the resection cavity by the direct intracranial drug delivery system.

In a preferred embodiment the composition for use in treating brain tumor in a subject comprises 5-aminolevulinic acid, wherein, in use, the composition is provided in an direct intracranial drug delivery system to a subject after at least partial surgical resection of the brain tumor in combination with a therapeutically effective amount of an artemisinin compound selected from **1a, 1b, 1c, 1d,** and **1e** or a pharmaceutically acceptable salt thereof, and at least one anti-glioblastoma drug selected from the group consisting of temozolomide, dexamethasone, lomustine, methotrexate, everolimus, carmustine, cyclophosphamide, cisplatin, carboplatin, 5-fluorouracil, triptolide, homoharringtonin, dactinomycin, doxorubicin, epirubicin, idarubicin, ribavirin, topotecan, flubendazole, itraconazole, vindesine sulfate, cerivastatin, vincristine, vinorebine, nisoldipine, deoxyadenosine, chloro-2'-deoxyadenosine, 5-nonyloxytryptamine, 2(1H)-pyrimidinone, pitavastatin, sertraline, irinotecan, clofazimine, tovorafenib, and docetaxel, wherein the artemisinin compound is daily administered orally or intravenously to the subject during the release of the therapeutically effective amount of the composition into the resection cavity by the direct intracranial drug delivery system, and wherein the therapeutically effective amount of at least one anti-glioblastoma drug is administered systemically to the subject during the release of the therapeutically effective amount of the composition into the resection cavity by the direct intracranial drug delivery system.

In a preferred embodiment the composition for use in treating brain tumor in a subject comprises 5-aminolevulinic acid, wherein, in use, the composition is provided in an direct intracranial drug delivery system to a subject after at least partial surgical resection of the brain tumor in combination with a therapeutically effective amount of an artemisinin compound selected from **1a, 1b, 1c, 1d,** and **1e** or a pharmaceutically acceptable salt thereof, and at least one anti-glioblastoma drug selected from the group consisting of temozolomide, lomustine, cisplatin, and 5 fluorouracil, wherein the artemisinin compound is daily administered orally or intravenously to the subject during the release of the therapeutically effective amount of the composition into the resection cavity by the direct intracranial drug delivery system, and wherein the therapeutically effective amount of at least one anti-glioblastoma drug is administered systemically to the subject during the release of the therapeutically effective amount of the composition into the resection cavity by the direct intracranial drug delivery system.

In a preferred embodiment the composition for use in treating brain tumor in a subject comprises 5-aminolevulinic acid, wherein, in use, the composition is provided in an direct intracranial drug delivery system to a subject after at least partial surgical resection of the brain tumor in combination with a therapeutically effective amount of an artemisinin compound selected from **1a, 1b, 1c, 1d,** and **1e** or a pharmaceutically acceptable salt thereof, and at least one anti-glioblastoma drug, wherein the direct intracranial drug delivery system releases a therapeutically effective amount of the composition into a resection cavity in a controlled-release manner, wherein the artemisinin compound is daily administered orally or intravenously to the subject during the release of the therapeutically effective amount of the composition into the resection cavity by the direct intracranial drug delivery system. and wherein the therapeutically effective amount of at least one anti-glioblastoma drug is administered systemically to the subject during the release of the therapeutically effective amount of the composition into the resection cavity by the direct intracranial drug delivery system in a range of 0.01 to 100 mg/kg per body weight per day.

Preferred, the dose of the anti-glioblastoma drug is administered orally daily is at least 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, 1.00, 1.05, 1.10, 1.15, 1.20, 1.25, 1.30, 1.35, 1.40, 1.45, 1.50, 2.00, 2.50, 3.00, 3.50, 4.00, 4.50, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5 or 15.0 mg/kg anti-glioblastoma drug per body weight per day.

Another aspect of the present invention is directed to the composition for use in treating brain tumor as described herein in combination with a radiotherapy, photodynamic therapy, immunotherapy, electromagnetic field therapy, hyperthermia therapy, chemotherapy, cancer immunotherapy and/or a cellular therapy such as Car-T and TIL. Preferred is a combination with chemotherapy, radiotherapy, and photodynamic therapy.

Reworded, in a preferred embodiment, the composition for use in treating brain tumor in a subject in combination with a radiotherapy, photodynamic therapy, immunotherapy, electromagnetic field therapy, hyperthermia therapy, chemotherapy, cancer immunotherapy, the composition comprises 5-aminolevulinic acid, wherein, in use, the composition is provided in an direct intracranial drug delivery system to a subject after at least partial surgical resection of the brain tumor, and wherein the direct intracranial drug delivery system releases a therapeutically effective amount of the composition into a resection cavity in a controlled-release manner, wherein the composition is continuously released into the resection cavity over 1 to 50 days after at least partial surgical resection of the brain tumor, and wherein the brain tumor is a glioblastoma multiforme.

In another embodiment, the composition is continuously released into the resection cavity over 1 to 50 days or 1 to 40 days or 1 to 30 days, more preferably over 2 to 30 days, more preferably over 3 to 30 days, more preferably over 4 to 30 days, more preferably over 5 to 30 days, and most preferably over 8 to 30 days.

Preferably, the composition is continuously released into the resection cavity over at least 4 days, more preferably at least 5 days, more preferably at least 6 days, more preferably at least 7 days, more preferably at least 8 days, more preferably at least 9 days, more preferably at least 10 days, more preferably at least 11 days, more preferably at least 12 days, more preferably at least 13 days, more preferably at least 14 days, more preferably at least 15 days, more preferably at least 16 days, more preferably at least 17 days, more preferably at least 18 days, more preferably at least 19 days, more preferably at least 20 days, more preferably at least 21 days, more preferably at least 22 days, more preferably at least 23 days, more preferably at least 24 days, more preferably at least 25 days, more preferably at least 26 days, more preferably at least 27 days, more preferably at least 28 days, more preferably at least 29 days, and most preferably at least 30 days after at least partial surgical resection of the brain tumor.

In another embodiment, the composition is continuously released into the resection cavity between 4 days to 30 days, more preferably between 5 days and 30 days, more preferably between 10 days and 30 days, more preferably between 15 days and 30 days, and most preferably between 20 days and 30 days after at least partial surgical resection of the brain tumor.

In another embodiment, the composition is continuously released into the resection cavity between 3 days to 15 days, more preferably between 4 days and 14 days, more preferably between 5 days and 13 days, more preferably between 6 days and 12 days, and most preferably between 7 days and 11 days after at least partial surgical resection of the brain tumor.

In another embodiment, the composition is continuously released into the resection cavity between 8 days to 20 days, more preferably between 9 days and 19 days, more preferably between 10 days and 18 days, more preferably between 11 days and 17 days, and most preferably between 12 days and 16 days after at least partial surgical resection of the brain tumor.

In another embodiment, the composition is continuously released into the resection cavity between 13 days to 25 days, more preferably between 14 days and 24 days, more preferably between 15 days and 23 days, more preferably between 16 days and 22 days, and most preferably between 17 days and 21 days after at least partial surgical resection of the brain tumor.

In another embodiment, the composition is continuously released into the resection cavity between 18 days to 30 days, more preferably between 19 days and 29 days, more preferably between 20 days and 28 days, more preferably between 21 days and 27 days, and most preferably between 22 days and 26 days after at least partial surgical resection of the brain tumor.

In another embodiment, the composition is continuously released into the resection cavity between 23 days to 35 days, more preferably between 24 days and 34 days, more preferably between 25 days and 33 days, more preferably between 26 days and 32 days, and most preferably between 27 days and 30 days after at least partial surgical resection of the brain tumor.

In a preferred embodiment, the composition for use in treating brain tumor in a subject in combination with a radiotherapy, photodynamic therapy, immunotherapy, electromagnetic field therapy, hyperthermia therapy, chemotherapy, cancer immunotherapy, the composition comprises 5-aminolevulinic acid, wherein, in use, the composition is provided in an direct intracranial drug delivery system to a subject after at least partial surgical resection of the brain tumor, and wherein the direct intracranial drug delivery system releases a therapeutically effective amount of the composition into a resection cavity in a controlled-release manner, wherein the 5-aminolevulinic acid in the composition is continuously released at a constant rate of 0.1 mg - 100 mg per day, and wherein the brain tumor is a glioblastoma multiforme.

Preferably, the 5-aminolevulinic acid in the composition is continuously released at a constant rate of 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day.

Preferably, the 5-aminolevulinic acid in the composition is continuously released at a constant rate of about 0.1 mg per day; more preferably at a constant rate of about 0.2 mg per day; more preferably at a constant rate of about 0.3 mg per day; more preferably at a constant rate of about 0.4 mg per day; more preferably at a constant rate of about 0.5 mg per day; more preferably at a constant rate of about 0.6 mg per day; more preferably at a constant rate of about 0.7 mg per day; more preferably at a constant rate of about 0.8 mg per day; more preferably at a constant rate of about 0.9 mg per day; more preferably at a constant rate of about 1.0 mg per day; more preferably at a constant rate of about 1.1 mg per day; more preferably at a constant rate of about 1.2 mg per day; more preferably at a constant rate of about 1.3 mg per day; more preferably at a constant rate of about 1.4 mg per day; more preferably at a constant rate of about 1.5 mg per day; more preferably at a constant rate of about 1.6 mg per day; more preferably at a constant rate of about 1.7 mg per day; more preferably at a constant rate of about 1.8 mg per day; more preferably at a constant rate of about 1.9 mg per day; more preferably at a constant rate of about 2.0 mg per day; more preferably at a constant rate of about 2.1 mg per day; more preferably at a constant rate of about 2.2 mg per day; more preferably at a constant rate of about 2.3 mg per day; more preferably at a constant rate of about 2.4 mg per day; more preferably at a constant rate of about 2.5 mg per day; more preferably at a constant rate of about 2.6 mg per day; more preferably at a constant rate of about 2.7 mg per day; more preferably at a constant rate of about 2.8 mg per day; more preferably at a constant rate of about 2.9 mg per day; more preferably at a constant rate of about 3.0 mg per day; more preferably at a constant rate of about 3.1 mg per day; more preferably at a constant rate of about 3.2 mg per day; more preferably at a constant rate of about 3.3 mg per day; more preferably at a constant rate of about 3.4 mg per day; more preferably at a constant rate of about 3.5 mg per day; more preferably at a constant rate of about 3.6 mg per day; more preferably at a constant rate of about 3.7 mg per day; more preferably at a constant rate of about 3.8 mg per day; more preferably at a constant rate of about 3.9 mg per day; more preferably at a constant rate of about 4.0 mg per day; more preferably at a constant rate of about 4.1 mg per day; more preferably at a constant rate of about 4.2 mg per day; more preferably at a constant rate of about 4.3 mg per day; more preferably at a constant rate of about 4.4 mg per day; more preferably at a constant rate of about 4.5 mg per day; more preferably at a constant rate of about 4.6 mg per day; more preferably at a constant rate of about 4.7 mg per day; more preferably at a constant rate of about 4.8 mg per day; more preferably at a constant rate of about 4.9 mg per day; more preferably at a constant rate of about 5.0 mg per day; more preferably at a constant rate of about 5.1 mg per day; more preferably at a constant rate of about 5.2 mg per day; more preferably at a constant rate of about 5.3 mg per day; more preferably at a constant rate of about 5.4 mg per day; more preferably at a constant rate of about 5.5 mg per day; more preferably at a constant rate of about 5.6 mg per day; more preferably at a constant rate of about 5.7 mg per day; more preferably at a constant rate of about 5.8 mg per day; more preferably at a constant rate of about 5.9 mg per day; more preferably at a constant rate of about 6.0 mg per day; more preferably at a constant rate of about 6.1 mg per day; more preferably at a constant rate of about 6.2 mg per day; more preferably at a constant rate of about 6.3 mg per day; more preferably at a constant rate of about 6.4 mg per day; more preferably at a constant rate of about 6.5 mg per day; more preferably at a constant rate of about 6.6 mg per day; more preferably at a constant rate of about 6.7 mg per day; more preferably at a constant rate of about 6.8 mg per day; more preferably at a constant rate of about 6.9 mg per day; more preferably at a constant rate of about 7.0 mg per day; more preferably at a constant rate of about 7.1 mg per day; more preferably at a constant rate of about 7.2 mg per day; more preferably at a constant rate of about 7.3 mg per day; more preferably at a constant rate of about 7.4 mg per day; more preferably at a constant rate of about 7.5 mg per day; more preferably at a constant rate of about 7.6 mg per day; more preferably at a constant rate of about 7.7 mg per day; more preferably at a constant rate of about 7.8 mg per day; more preferably at a constant rate of about 7.9 mg per day; more preferably at a constant rate of about 8.0 mg per day; more preferably at a constant rate of about 8.1 mg per day; more preferably at a constant rate of about 8.2 mg per day; more preferably at a constant rate of about 8.3 mg per day; more preferably at a constant rate of about 8.4 mg per day; more preferably at a constant rate of about 8.5 mg per day; more preferably at a constant rate of about 8.6 mg per day; more preferably at a constant rate of about 8.7 mg per day; more preferably at a constant rate of about 8.8 mg per day; more preferably at a constant rate of about 8.9 mg per day; more preferably at a constant rate of about 9.0 mg per day; more preferably at a constant rate of about 9.1 mg per day; more preferably at a constant rate of about 9.2 mg per day; more preferably at a constant rate of about 9.3 mg per day; more preferably at a constant rate of about 9.4 mg per day; more preferably at a constant rate of about 9.5 mg per day; more preferably at a constant rate of about 9.6 mg per day; more preferably at a constant rate of about 9.7 mg per day; more preferably at a constant rate of about 9.8 mg per day; more preferably at a constant rate of about 9.9 mg per day; and most preferably at a constant rate of about 10.0 mg per day.

In a preferred embodiment, the composition for use in treating brain tumor in a subject in combination with a radiotherapy, photodynamic therapy, immunotherapy, electromagnetic field therapy, hyperthermia therapy, chemotherapy, cancer immunotherapy, the composition comprises 5-aminolevulinic acid, wherein, in use, the composition is provided in an direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity over 1 to 30 days, preferably 7 to 30 days, more preferably 12 to 29 days, more preferably 14 to 28 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the brain tumor is a glioblastoma multiforme.

In a preferred embodiment, the composition for use in treating brain tumor in a subject in combination with a radiotherapy, photodynamic therapy, immunotherapy, electromagnetic field therapy, hyperthermia therapy, chemotherapy, cancer immunotherapy, the composition comprises 5-aminolevulinic acid, wherein, in use, the composition is provided in an direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 3 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the brain tumor is a glioblastoma multiforme.

In a preferred embodiment, the composition for use in treating brain tumor in a subject in combination with a radiotherapy, photodynamic therapy, immunotherapy, electromagnetic field therapy, hyperthermia therapy, chemotherapy, cancer immunotherapy, the composition comprises 5-aminolevulinic acid, wherein, in use, the composition is provided in an direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 10 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the brain tumor is a glioblastoma multiforme.

In a preferred embodiment, the composition for use in treating brain tumor in a subject in combination with a radiotherapy, photodynamic therapy, immunotherapy, electromagnetic field therapy, hyperthermia therapy, chemotherapy, cancer immunotherapy, the composition comprises 5-aminolevulinic acid, wherein, in use, the composition is provided in an direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 20 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the brain tumor is a glioblastoma multiforme.

In a preferred embodiment, the composition for use in treating brain tumor in a subject in combination with a radiotherapy, photodynamic therapy, immunotherapy, electromagnetic field therapy, hyperthermia therapy, chemotherapy, cancer immunotherapy, the composition comprises 5-aminolevulinic acid, wherein, in use, the composition is provided in an direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 25 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the brain tumor is a glioblastoma multiforme.

In a preferred embodiment, the composition for use in treating brain tumor in a subject in combination with a radiotherapy, photodynamic therapy, immunotherapy, electromagnetic field therapy, hyperthermia therapy, chemotherapy, cancer immunotherapy, the composition comprises 5-aminolevulinic acid, wherein, in use, the composition is provided in an direct intracranial drug delivery system to a subject after at least partial surgical resection of the brain tumor, and wherein the direct intracranial drug delivery system releases a therapeutically effective amount of the composition into a resection cavity in a controlled-release manner, and wherein the composition is continuously released into the resection cavity for at least 30 days after at least partial surgical resection of the brain tumor, wherein the 5-aminolevulinic acid in the composition is continuously released at a constant rate of about 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, more preferably at a constant rate of 0.5 mg - 10 mg per day, and most preferably at a constant rate of 2 mg - 20 mg per day, and wherein the brain tumor is a glioblastoma multiforme.

Preferably, the composition for use in treating brain tumor in a subject as described herein is combined with a radiotherapy or photodynamic therapy.

Thus, a further aspect of the present invention is directed to a composition for use in treating brain tumor in a subject as described herein in combination with a radiotherapy or photodynamic therapy and with a therapeutically effective amount of an artemisinin compound selected from **1a, 1b, 1c, 1d,** and **1e** or a pharmaceutically acceptable salt thereof, wherein the artemisinin compound is daily administered orally or intravenously to the subject during the release of the therapeutically effective amount of the composition into the resection cavity by the direct intracranial drug delivery system.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with a radiotherapy or photodynamic therapy and with artesunate (**1e**)., wherein the direct intracranial drug delivery system releases a therapeutically effective amount of the composition into a resection cavity in a controlled-release manner, and wherein the artesunate (**1e**) is daily administered orally or intravenously to the subject during the release of the therapeutically effective amount of the composition into the resection cavity by the direct intracranial drug delivery system.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with a radiotherapy or photodynamic therapy and with an artemisinin compound selected from **1a, 1b, 1c, 1d,** and **1e** or a pharmaceutically acceptable salt thereof, wherein the direct intracranial drug delivery system releases a therapeutically effective amount of the composition into a resection cavity in a controlled-release manner, wherein the artemisinin compound is administered daily administered orally or intravenously to the subject during the release of the therapeutically effective amount of the composition into the resection cavity by the direct intracranial drug delivery system, and wherein the daily dose of the artemisinin compound is in a range of 0.5 mg artemisinin compound per kg body weight to 10 mg artemisinin compound per kg body weight.

Preferably, the daily dose of the artemisinin compound is between about 0.1 mg and 100 mg artemisinin compound per kg body weight, more preferably about 0.2 mg and 80 mg artemisinin compound per kg body weight, more preferably about 0.3 mg and 50 mg artemisinin compound per kg body weight, more preferably about 0.4 mg and 25 mg artemisinin compound per kg body weight, more preferably about 0.5 mg and 10 mg artemisinin compound per kg body weight, more preferably about 1 mg and 8 mg artemisinin compound per kg body weight, more preferably about 2 mg and 6 mg artemisinin compound per kg body weight, and most preferably about 3 mg artemisinin compound per kg body weight.

Preferably, the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with a radiotherapy or photodynamic therapy and with an artemisinin compound selected from **1a, 1b, 1c, 1d,** and **1e** or a pharmaceutically acceptable salt thereof, wherein the direct intracranial drug delivery system releases a therapeutically effective amount of the composition into a resection cavity in a controlled-release manner, wherein the artemisinin compound is administered daily administered orally or intravenously to the subject during the release of the therapeutically effective amount of the composition into the resection cavity by the direct intracranial drug delivery system, and wherein the total daily dose of the artemisinin compound is in a range of 100 mg to 300 mg, preferably 150 mg to 250 mg, preferably 180 mg to 220 mg, and most preferably about 200 mg.

Preferably, the total daily dose of the artemisinin compound is 100 mg, more preferably 105 mg, more preferably 110 mg, more preferably 115 mg, more preferably 120 mg, more preferably 125 mg, more preferably 130 mg, more preferably 135 mg, more preferably 140 mg, more preferably 145 mg, more preferably 150 mg, more preferably 155 mg, more preferably 160 mg, more preferably 165 mg, more preferably 170 mg, more preferably 175 mg, more preferably 180 mg, more preferably 185 mg, more preferably 190 mg, more preferably 195 mg, and most preferably 200 mg.

Preferably, the total daily dose of the artemisinin compound is 300 mg, more preferably 295 mg, more preferably 290 mg, more preferably 285 mg, more preferably 280 mg, more preferably 275 mg, more preferably 270 mg, more preferably 265 mg, more preferably 260 mg, more preferably 255 mg, more preferably 250 mg, more preferably 245 mg, more preferably 240 mg, more preferably 235 mg, more preferably 230 mg, more preferably 225 mg, more preferably 220 mg, more preferably 215 mg, more preferably 210 mg, more preferably 205 mg, and most preferably 200 mg.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with a radiotherapy or photodynamic therapy and with an artemisinin compound selected from **1a, 1b, 1c, 1d,** and **1e** or a pharmaceutically acceptable salt thereof, wherein the direct intracranial drug delivery system releases a therapeutically effective amount of the composition into a resection cavity in a controlled-release manner, wherein the artemisinin compound is administered daily administered orally or intravenously to the subject during the release of the therapeutically effective amount of the composition into the resection cavity by the direct intracranial drug delivery system, and wherein a dose of the artemisinin compound of 100 mg is administered twice daily to the subject.

Preferably, the artemisinin compound is administered orally daily over 1 to 50 days or 1 to 40 days or 1 to 30 days, more preferably over 2 to 30 days, more preferably over 3 to 30 days, more preferably over 4 to 30 days, preferably over 5 to 30 days, and more preferably over 8 to 30 days, more preferably over 10 to 29 days, and most preferably over 14 to 28 days.

### Description of the Figures

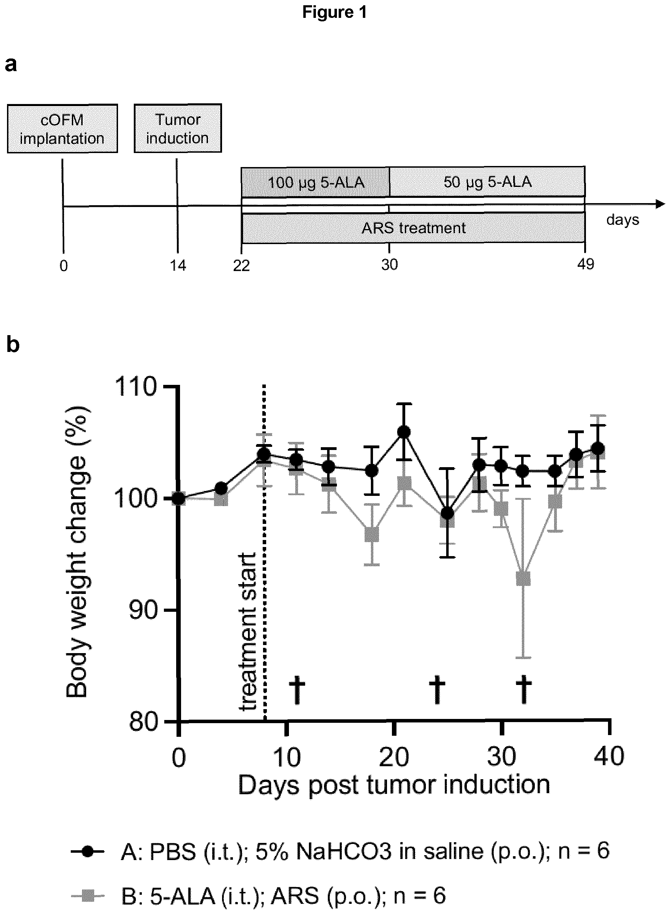

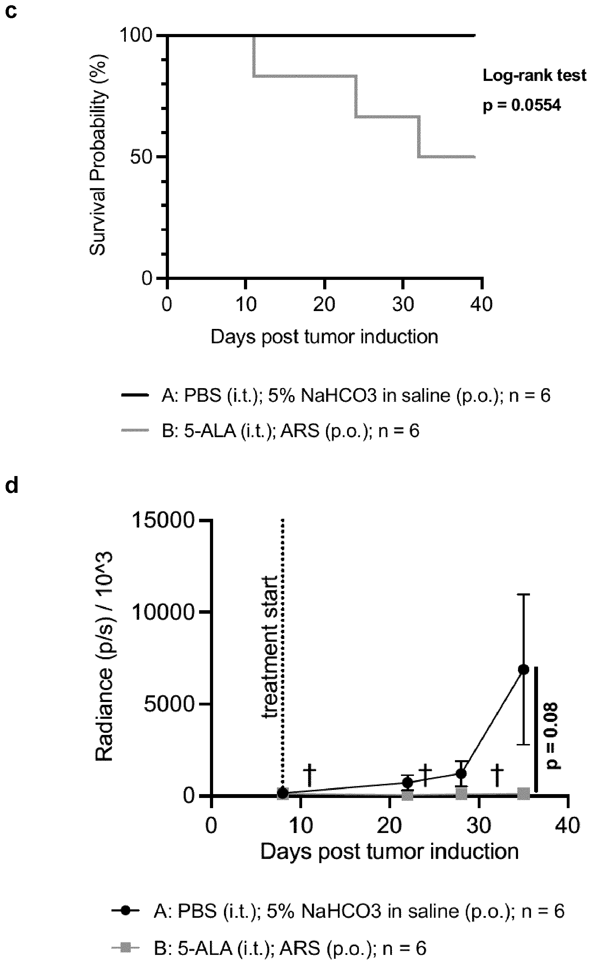

- **Figure 1**: shows an efficacy study using an orthothopic patient derived xenograft (PDX) model in mice. Patient-derived glioblastoma cells were implanted into the mouse brain via a cerebral open flow microperfusion (cOFM) device. **a,** Schematic representation of the study timeline. 14 days after cOFM implantation VBT529 (patient derived glioblastoma cell line) were implanted into the mouse brain via a cerebral open flow microperfusion (cOFM) device. 5-ALA bolus treatments were performed via the cOFM device and ARS was administered per oral (5x per week). **b,** % body weight changes of mice receiving solvents (PBS plus 5% NaHCO3 in saline) or 5-ALA plus ARS. †: premature termination of group B mice due to unexpected adverse effects. Data are presented as mean ± SEM; 2-way ANOVA followed by Bonferroni's multiple comparisons test. **c,** Kaplan-Meier survival curves of control and 5-ALA/ARS treated mice. Data analysis was performed using Log-rank (Mantel-Cox) test. **d,** Quantification of VBT529 brain tumor luminescence signals. †: premature termination of group B mice due to unexpected adverse effects. Data are presented as mean ± SEM; 2-way ANOVA followed by Bonferroni's multiple comparisons test.

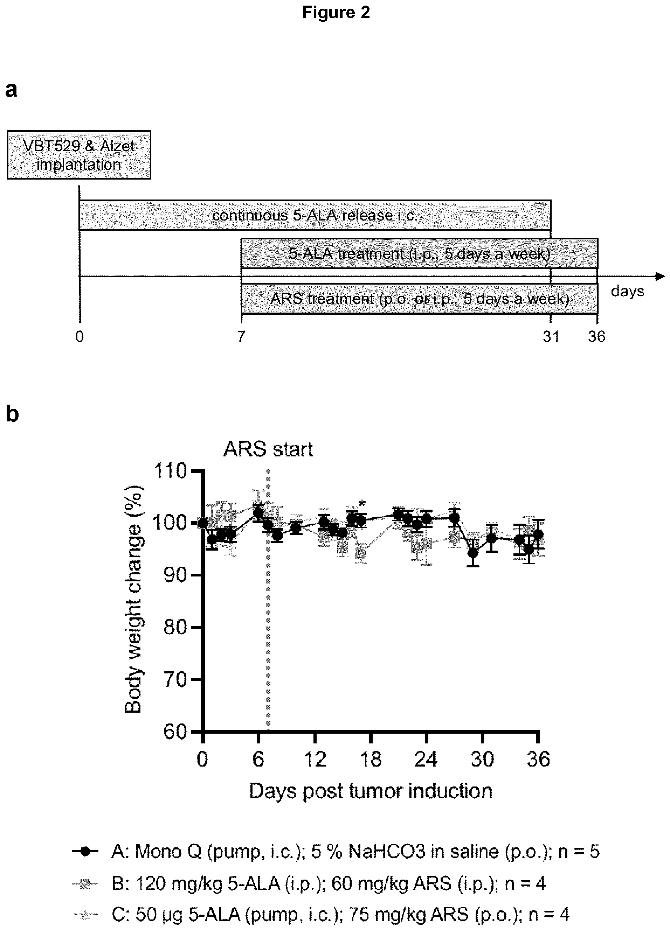

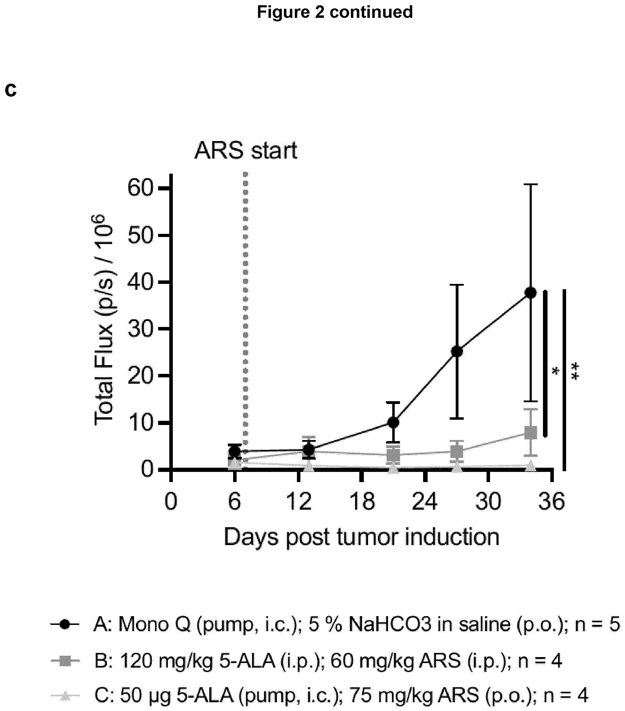

- **Figure 2**: shows an efficacy study using an orthothopic patient derived xenograft (PDX) model in mice comparing i.p. bolus treatments (5-ALA plus ARS) to an intracerebral slow-release 5-ALA treatment (via Alzet osmotic pump) combined with oral ARS. **a,** Schematic representation of the study timeline. 7 days after VBT529 (patient derived glioblastoma cell line) and Alzet pump implantation, ARS treatment (5 times a week) started. **b,** % body weight changes of mice receiving solvents or 5-ALA plus ARS. Data are presented as mean ± SEM; *p < 0,05 (Group A vs Group B and Group B vs. Group C); 2-way ANOVA followed by Bonferroni's multiple comparisons test. **c,** Quantification of VBT529 brain tumor luminescence signals. Data are presented as mean ± SEM; *p < 0,05; **p < 0,01; 2-way ANOVA followed by Bonferroni's multiple comparisons test.

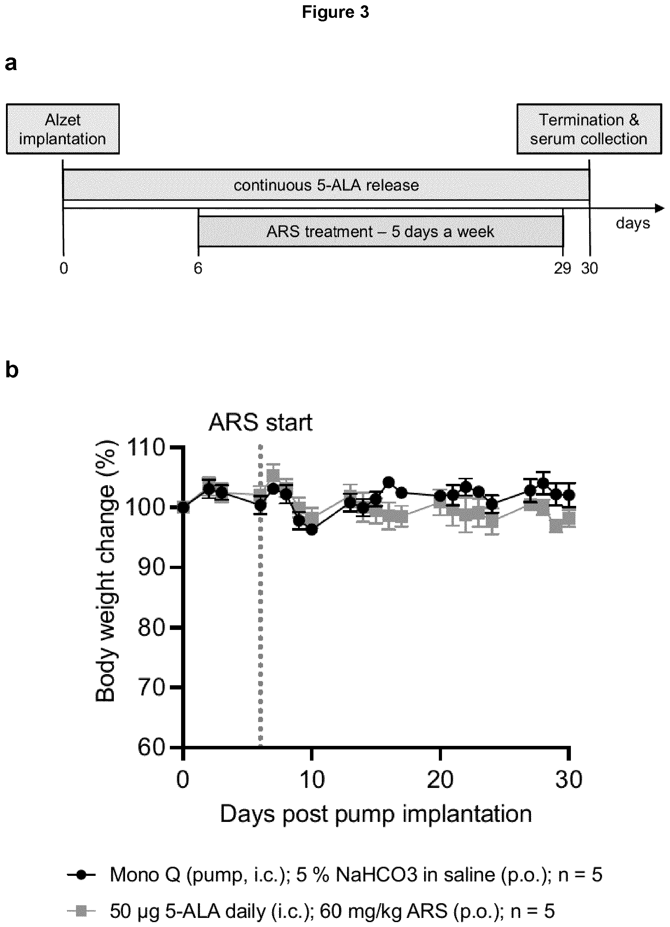

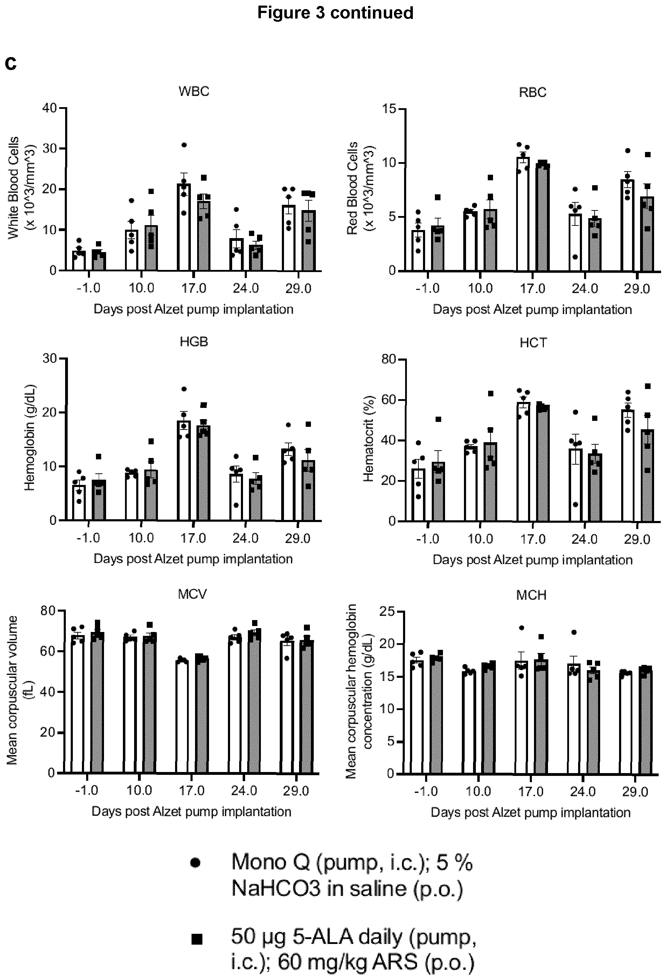

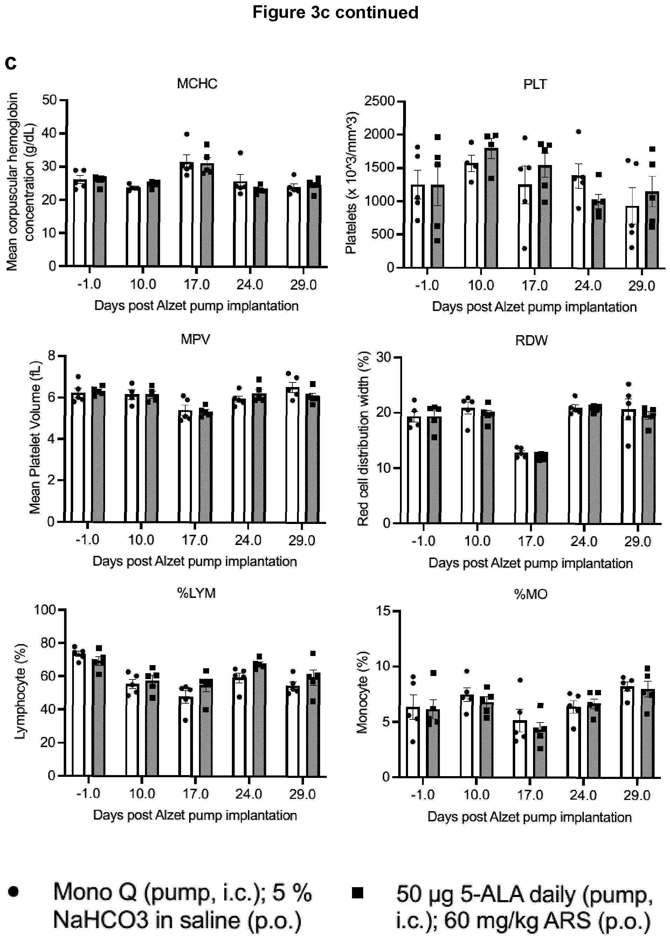

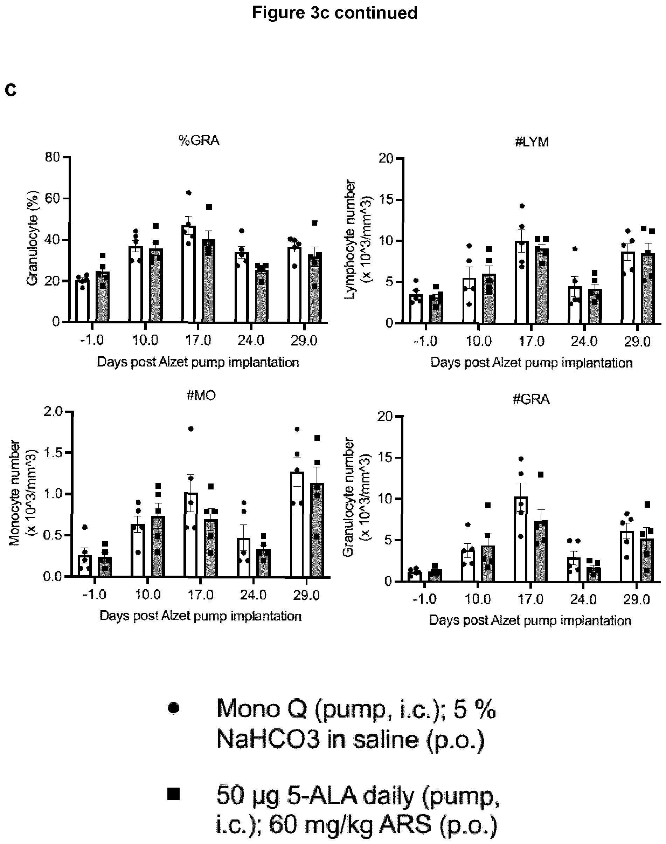

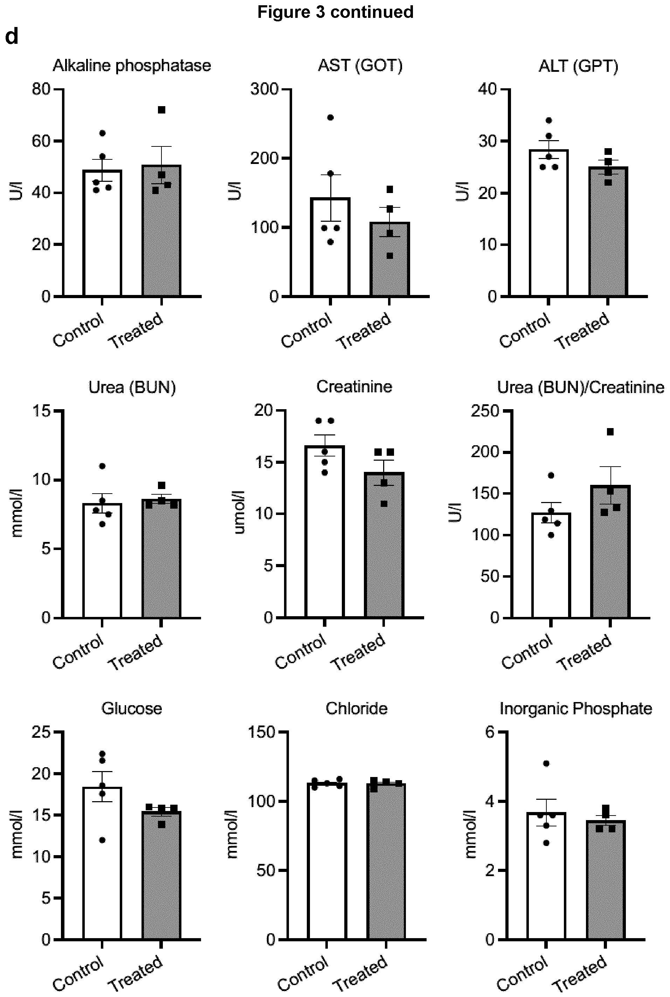

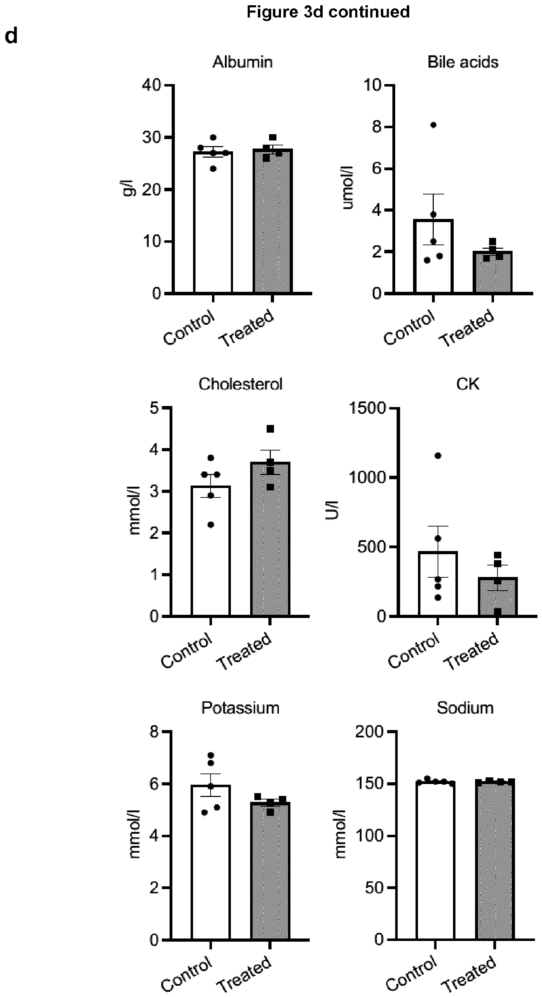

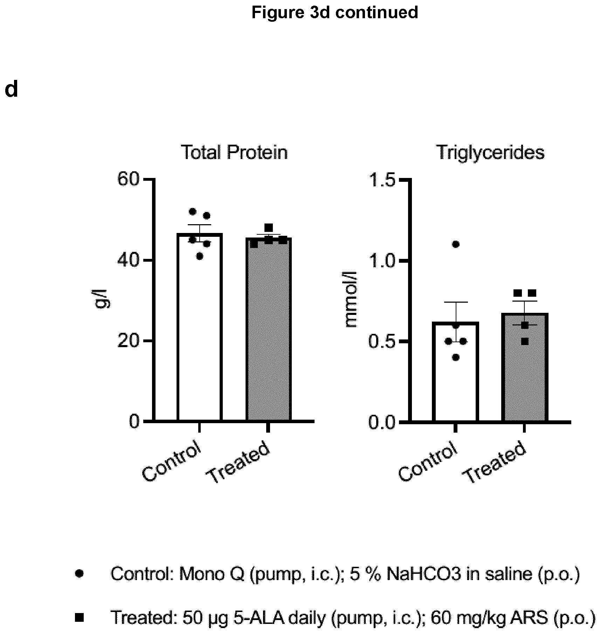

- **Figure 3**: shows a toxicity study in mice utilizing Alzet osmotic pumps connected to a brain infusion cannula to deliver different doses of 5-ALA directly into the brain via a constant slow-release mechanism. ARS was administered per oral. a, Schematic representation of the study timeline. 6 days after Alzet pump implantation, ARS treatment (5 times a week) started. **b,** % body weight changes of mice receiving solvents or 5-ALA plus ARS. Data are presented as mean ± SEM; 2-way ANOVA followed by Bonferroni's multiple comparisons test. **c,** Blood cell counts were performed 1 day before and 10-, 17-, 24- and 29-days post Alzet pump implantation. Each graph represents a distinct blood cell type/parameter as indicated. Data are presented as mean ± SEM; Student's unpaired t-test with Bonferroni's multiple comparisons test. **d,** Mouse sera were harvested 30 days post Alzet pump implantation (23 days of ARS treatment) and an expanded mouse serum tox analysis was performed. Each graph represents a distinct serum parameter as indicated. Data are presented as mean ± SEM; Student's unpaired t-test with Bonferroni's multiple comparisons test.

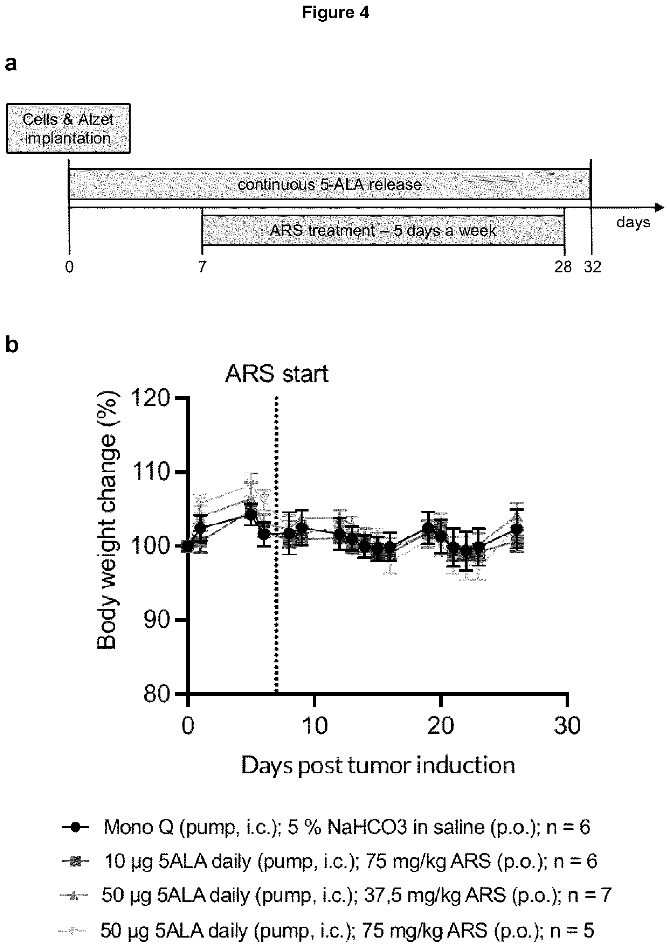

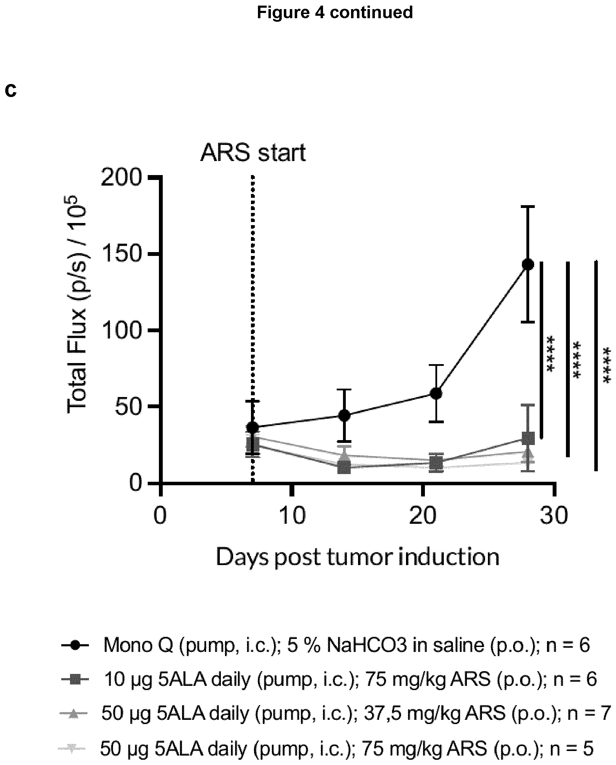

- **Figure 4**: shows an efficacy study using an orthothopic patient derived xenograft (PDX) model in mice using different doses of intracerebral slow-release 5-ALA plus oral ARS treatment. **a,** Schematic representation of the study timeline. 7 days after VBT529 (patient derived glioblastoma cell line) and Alzet pump implantation, ARS treatment (5 times a week) started. b, % body weight changes of mice receiving solvents or different doses of 5-ALA plus ARS. Data are presented as mean ± SEM; 2-way ANOVA followed by Bonferroni's multiple comparisons test. c, Quantification of VBT529 brain tumor luminescence signals. Data are presented as mean ± SEM; ****p < 0,0001; 2-way ANOVA followed by Bonferroni's multiple comparisons test.

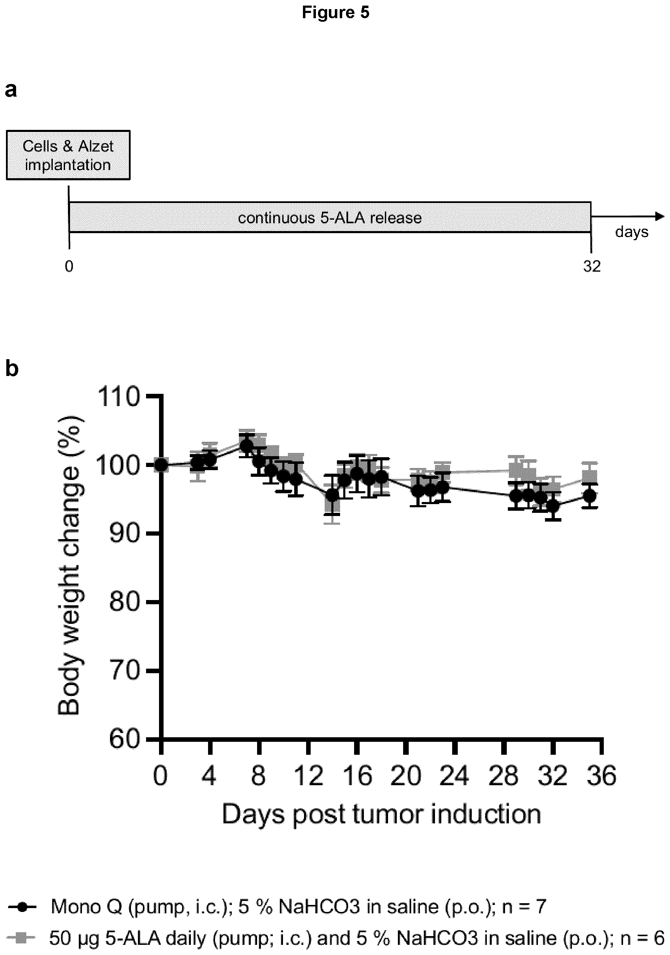

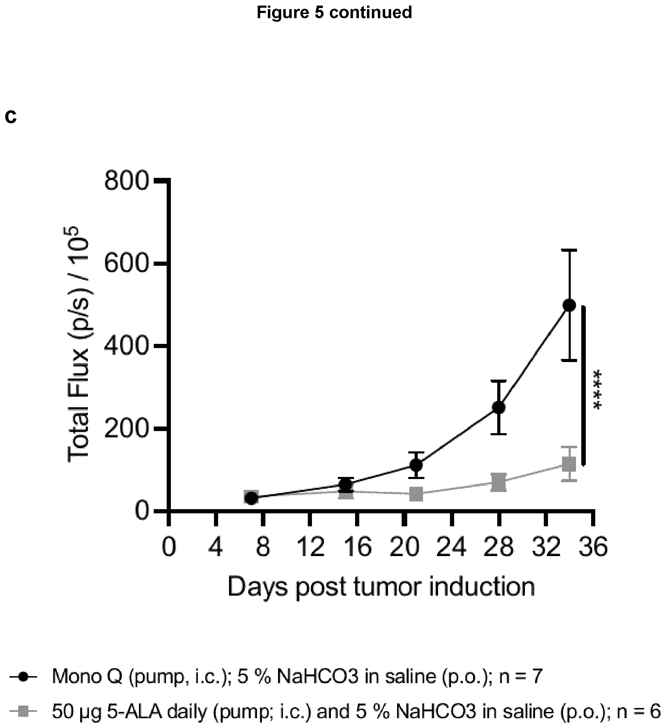

- **Figure 5**: shows an efficacy study using an orthothopic patient derived xenograft (PDX) model in mice utilizing Alzet osmotic pumps to deliver 5-ALA directly into the brain via a constant slow-release mechanism. **a,** Schematic representation of the study timeline. **b,** % body weight changes of mice receiving slow-release 5-ALA treatment. Data are presented as mean ± SEM; 2-way ANOVA followed by Bonferroni's multiple comparisons test. c, Quantification of VBT529 brain tumor luminescence signals. Data are presented as mean ± SEM; ****p < 0,0001; 2-way ANOVA followed by Bonferroni's multiple comparisons test.

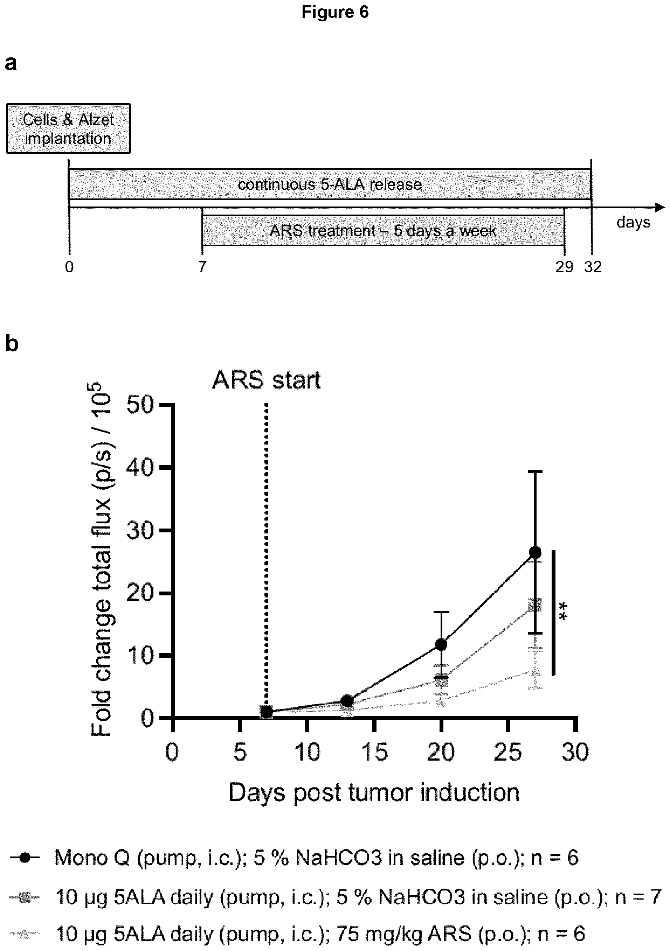

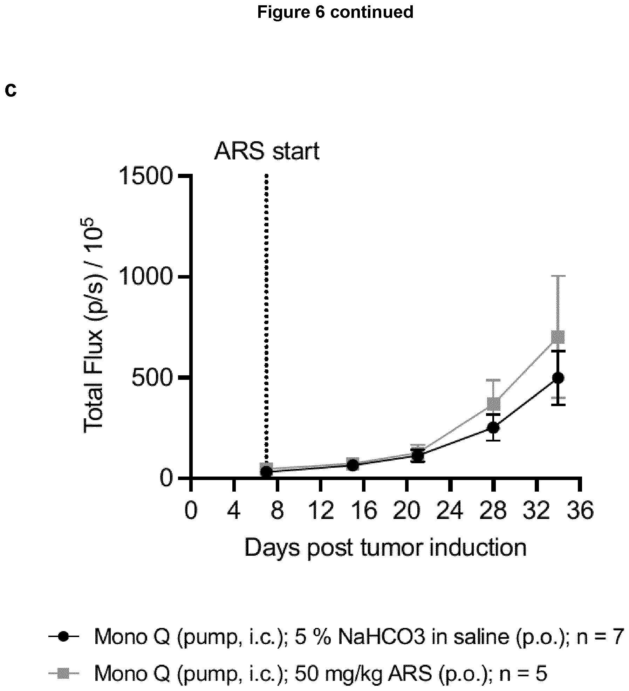

- **Figure 6**: shows an efficacy study using an orthothopic patient derived xenograft (PDX) model in mice utilizing Alzet osmotic pumps to deliver 5-ALA directly into the brain via a constant slow-release mechanism. **a,** Schematic representation of the study timeline. 7 days after VBT529 (patient derived glioblastoma cell line) and Alzet pump implantation, ARS treatment (5 times a week) started. **b** and **c,** Quantification of VBT529 brain tumor luminescence signals. Data are presented as mean ± SEM; **p < 0,01; 2-way ANOVA followed by Bonferroni's multiple comparisons test.

The following examples are included to demonstrate preferred embodiments of the invention. It should be appreciated by those skilled in the art that the techniques disclosed in the examples, which follow represent techniques discovered by the inventor to function well in the practice of the invention, and thus can be considered to constitute preferred modes for its practice. However, those skilled in the art should, in light of the present disclosure, appreciate that many changes can be made in the specific embodiments, which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Examples

### Materials and Methods

**Patient-derived xenograft (PDX) model.** Patients with malignant brain tumors and/or their legal representatives treated at the Medical University of Vienna gave preoperative informed consent to participate in the study in all cases. The study was approved by the local institutional review board (IRB) of the Medical University of Vienna (EK Nr. 1244/2016, EK Nr: 1616/2020) according to the guidelines of the Helsinki Declaration developed by the World Medical Association and the Department of Health and Human Services Belmont Report. The patient-derived brain tumor model VBT529 was derived from an aggressive glioblastoma and established at the Medical University of Vienna, Austria. VBT529 cells were classified as BSL 1 being tested negative at IDEXX for Hepatitis B/C and HIV 1/2. Cells were transduced with a lentiviral vector containing Luc2 (Luciferase), iRFP (infra-red fluorescent protein) and BlaS (blasticidin resistance gene). Following a two-week selection period on Blasticidin cells were cultivated in RPMI-1640 supplemented with 10 % fetal bovine serum, 1 % L-Glutamin and 1 % PenStep and grown at 37°C and 5% CO₂.

Mice were maintained, examined, and euthanized in accordance with institutional animal care guidelines and ethical animal license protocols approved by the legal authorities. Experimental animals were purchased from Charles River and housed at the Institute of Molecular Biotechnology (IMBA, Vienna, Austria) or the division of Biomedical Research (Medical University of Graz, Austria), in a 12-h light/dark cycle, with food and water ad libitum. Only female Crl:NU(NCr)-Foxn1<nu> (Nude) mice purchased from Charles River group housed in individually ventilated cages were used for all experiments described. Animals were randomly assigned to experimental groups and checked daily by veterinary staff. For orthotopic PDX, mice were anesthetized and restrained in a stereotaxic frame. The body temperature was monitored with a rectal thermometer and kept constant at 36°C by a heating pad. The skull was exposed, cleaned, and a small hole was drilled. 1,5 × 10⁵ VBT529 cells were injected at coordinates: AP-1, ML+2, DV-3. To prevent backflow, the needle was left at the injection site for 6 min after the injection was finished. Mice were left to recover for at least 1 week after the surgery and during that time their drinking water was supplied with Baytril (Bayer) and Rimadyl (Pfizer). 5-ALA (5-aminolevulinic acid hydrochloride, HY-N0305, MedchemExpress) was dissolved in water, and ARS (Artesunate, PHR2573, Sigma) was dissolved in 5 % NaHCO₃ (8551.1, Roth) and 0.9 % NaCl (3570130, B. Braun). 5-ALA and Artesunate were administered at the concentrations and timepoints indicated in the figure legends. Tumor growth was monitored using the IVIS Spectrum machine (Caliper Life Sciences). Mice were anesthetized using an isoflurane chamber. Visualization of the tumor cells expressing luciferase was achieved by retroorbital injection of 100 µL D-luciferin (15 mg/mL dissolved in PBS; Goldbio, LUCK-1G) and luminescence data were analyzed using the Living Image software.

**Patient-derived xenograft (PDX) model using cOFM probe.** Animals were anesthetized by inhalation narcosis (Isoflurane, 3 % in 1.9 L/h O₂). Pain treatment was maintained by Fentanyl (5 µg/kg, i.p.) and Carprofen (5 mg/kg, s.c.). After shaving the surgical area, animals were placed in stereotaxic equipment and the skull was exposed by a midline incision. Three holes for probe implantation (coordinates: AP 0,6/ML 2/ DV 4) and one hole for the anchor screw were drilled. Dura mater was opened using a hypodermic needle (30 Ga) and the cOFM (cerebral open flow microperfusion) probe was lowered into the brain at approx. 1 mm/min using the stereotaxic equipment. The surgical area was covered by dental cement. Finally, animals were treated with the antibiotic Enrofloacin (7.5 mg/kg, s.c.) and returned to their home cage for recovery.

VBT529 cells were injected on day 14 post cOFM probe implantation. Mice were anesthetized using an isoflurane chamber. A cOFM infusion insert was connected to the pump tubing and the cell suspension was aspired through the cOFM infusion insert (1,5 × 10⁵ VBT529 cells in 3 µl). The cOFM healing dummy was replaced with the cOFM infusion insert and cells were injected at a flow rate of 1 µl/min using a precision pump. After complete infusion of 3 µl cell suspension, the cOFM infusion insert was kept in place for at least 1 additional minute to avoid back flow out of the cOFM cannula after disconnection of the cOFM infusion insert. The cOFM infusion insert was replaced with the cOFM healing dummy and animals were transferred back to their home cage. Animals were closely monitored until wake-up and body temperature was maintained using a warming blanket.

For intratumoral drug application, animals were anesthetized using an isoflurane chamber. Compounds were aspired through the cOFM infusion insert. The cOFM healing dummy was replaced with the cOFM infusion insert and compounds were infused at a flow rate of 1 µl/min using a precision pump. After complete infusion the cOFM infusion insert was kept in place for 1 additional minute to avoid back flow. The cOFM infusion insert was replaced by the cOFM healing dummy and animals were transferred back to their home cage. Tumor growth was monitored using the LAGO device (spectral instruments imaging, Bruker). Mice were anesthetized using an isoflurane chamber. Visualization of the tumor cells expressing luciferase was achieved by i.p. injection of 100 µL D-luciferin (15 mg/mL dissolved in PBS; Goldbio, LUCK-1G) and luminescence data were acquired.

**Blood cell counts.** Mouse blood samples were collected into micro tubes containing 1,6 mg/mL EDTA (41.1504.015, Sarstedt) and blood cell counts were performed on scil Vet abc animal blood counter.

**Serum Analysis IDEXX.** Mouse blood samples were collected into Microtainer SST blood collection tubes (365968, Becton Dickinson) and serum samples were sent to IDEXX BioAnalytics (70806 Kornwestheim, Germany) for rodent expanded tox panel analysis.

**Statistical analysis.** All data are expressed as mean ± standard error of the mean (SEM). Statistical significance was tested by 2-way ANOVA followed by Bonferroni's post hoc test; Mantel-Cox test or Student's unpaired t-test followed by Bonferroni's post hoc test. All figures and mouse statistical analyses were generated using Prism 10 (GraphPad). Details of the statistical tests used are stated in the figure legends. In all figures, statistical significance is represented as *p < 0.05, **p < 0.01, ****p < 0.0001.

### Example 1: Intracranial 5-aminolevulinic acid (5-ALA) bolus treatments combined with oral Artesunate (ARS) show a positive trend on tumor growth restriction but lead to systemic adverse events.

The inventors sought to identify an application scheme of 5-aminolevulinic acid (5-ALA) in combination with Artesunate (ARS) for the effective and safe treatment of brain tumors. Therefore, mice were equipped with a cerebral open flow microperfusion (cOFM) device, which allows frequent intracranial injections. Next, patient derived glioblastoma cells (VBT529) were implanted into the mouse brain via the cOFM device (Fig. 1a). 8 days post implantation, animals were treated with an intracranial 5-ALA bolus (via cOFM device) combined with oral ARS treatment on every weekday for 4 consecutive weeks (Fig. 1a). Body weight and tumor volume (radiance of LAGO imaging) were monitored. While the treatment had a positive, but non-significant antineoplastic effect, the intracranial 5-ALA bolus had to be reduced from 100 to 50 µg per day and the treatment showed adverse effects on body weight and animal survival (Fig. 1b-d).

### Example 2: Constant intracranial 5-ALA release combined with oral ARS shows strong antitumor activity and is well tolerated.

To improve efficacy and reduce adverse side effects the inventors set out to study a constant low-dose intracranial 5-ALA release method in mouse xenograft models. Patient derived tumor cells (VBT529) were injected into the brain of nude mice and a slow-release osmotic pump (Alzet) loaded with 5-ALA was installed to allow constant release of 5-ALA into the brain over a period of 1 month (Fig. 2a). The continuous 5-ALA treatment was combined with per oral ARS dosing on weekdays and body weight as well as tumor volume were monitored. A third group was treated with 5-ALA and ARS systemically (i.p.) on weekdays for 4 weeks at doses close to the highest tolerated systemic exposures. Both treatment regiments showed strong antineoplastic activity and had no impact on body weights (Fig. 2b, c). Surprisingly, constant intracranial 5-ALA combined with oral ARS treatment showed the strongest antineoplastic effects despite the very low 5-ALA dose of 50 µg per day compared to 120 mg/kg in the systemic group.

### Example 3: The combination of constant intracranial 5-ALA release combined with oral Artesunate shows no effect on body weight, blood cell parameters and serum tox parameters in healthy mice.

The combination of constant intracranial 5-ALA release combined with oral ARS was tested in healthy animals where no tumor cells were implanted. Animals were treated for 1 month with a constant intracranial 5-ALA release (via Alzet osmotic pump) combined with 23 days of per oral ARS treatment on weekdays (Fig. 3a). Body weight was monitored daily, blood samples for blood cell parameters were collected before- and on days 10, 17, 24, and 29 post treatment start, and serum was analyzed at the end of the study. Continuous intracranial 5-ALA treatment combined with per oral ARS had no effect on body weight, blood cell parameters, and toxicological markers assessed in serum demonstrating the safety of this new application scheme (Fig. 3b-d).

### Example 4: Different intracranial constant release 5-ALA and oral ARS dose combinations prevent tumor growth in mice, while being well tolerated.

Patient derived tumor cells (VBT529) were injected into the brain of nude mice and slow-release osmotic pumps (Alzet) loaded with 5-ALA were installed to allow constant release of 5-ALA into the mouse brain over a period of 1 month (Fig. 4a). The continuous 5-ALA treatment was combined with per oral ARS dosing on weekdays for 3 weeks, testing two 5-ALA and two ARS concentrations. Body weight as well as tumor volume were monitored. All tested 5-ALA/ARS combinations showed strong antineoplastic activity while not effecting animal body weights (Fig. 4b, c). Surprisingly, even the extremely low 5-ALA dose of 10 µg per day significantly reduced tumor growth in combination with oral ARS treatment.

### Example 5: Constant intracranial 5-ALA release alone significantly reduces brain tumor growth.

To assess the antineoplastic activity of 5-ALA alone, patient derived tumor cells (VBT529) were injected into the brain of nude mice and a slow-release osmotic pump (Alzet) loaded with 5-ALA was installed to allow constant release of 5-ALA into the brain over a period of 1 month (Fig. 5a). Body weight as well as tumor volume were monitored. The body weight remained unaffected by the 5-ALA treatment, but tumor growth was significantly reduced indicating that constant intracranial 5-ALA release alone has antineoplastic activity (Fig. 5b, c).

### Example 6: Constant intracranial 5-ALA release alone slightly reduces tumor growth, while the combination with oral ARS shows strong antineoplastic activity.

Patient derived tumor cells (VBT529) were injected into the brain of nude mice and slow-release osmotic pumps (Alzet) loaded with 5-ALA were installed to allow constant release of 5-ALA into the brain over a period of 1 month (Fig. 6a). In one group, the 5-ALA treatment was combined with per oral Artesunate treatment. In parallel, the effect of only ARS treatment on tumor growth was tested. Constant, low-dose intracranial 5-ALA release showed mild, but non-significant antitumor activity, which was boosted by the combination with per oral ARS treatment (Fig. 6b). ARS treatment alone did not limit tumor growth in this setup, highlighting the synergistic effects of the combination of constant 5-ALA release with per oral ARS (Fig. 6c).

## Claims

1. A composition for use in treating brain tumor in a subject, the composition comprising 5-aminolevulinic acid, wherein, in use, the composition is provided by a direct intracranial drug delivery system to a subject after at least partial surgical resection of the brain tumor, and wherein the direct intracranial drug delivery system releases a therapeutically effective amount of the composition into a resection cavity in a controlled-release manner.

2. The composition for use according to claim 1, wherein the composition is continuously released into the resection cavity over 4 to 30 days.

3. The composition for use according to claim 1 or 2, wherein the 5-aminolevulinic acid in the composition is continuously released at a constant rate of 0.1 mg - 100 mg per day.

4. The composition for use according to any one of claims 1 to 3, wherein the brain tumor is a glioma, preferably a glioblastoma multiforme.

5. The composition for use according to any one of claims 1 to 4, wherein the direct intracranial drug delivery system is an implantable system comprising
a) a core comprising:
5-aminolevulinic acid and a core polymer,
wherein the core polymer is selected from the group consisting of poly(lactic acid), poly(ethylene oxide), poly(ethylene glycol), and poly (lactic-co-glycolic acid); and
b) a shell consisting of an upper membrane, a spacer, and a lower membrane, wherein the upper membrane, the spacer, and the lower membrane each comprise a shell polymer, wherein the shell polymer is selected from polyl(actic acid);
**characterized in that**
the shell polymer is in a range from 20 wt.% to 60 wt.% based on the total weight of the implantable system; and
when the core polymer is poly(lactic acid), poly(ethylene glycol), or poly(ethylene oxide), the core contains 5-aminolevulinic acid in a range from 25 wt.% to 45 wt. %, and the core polymer in a range from 75 wt.% to 55 wt.% based on the total weight of the core; or
when the core polymer is poly(lactic-co-glycolic acid), the core contains 5-aminolevulinic acid in a range from 65 wt.% to 75 wt.% and the core polymer in a range from 25 wt.% to 35 wt.% based on the total weight of the core.

6. The composition for use according to any one of claims 1 to 4, wherein the system comprises
a) a core consisting of:
65 wt.% to 75 wt.% of 5-aminolevulinic acid, and 35 wt.% to 25 wt.% of poly (lactic-co-glycolic acid), based on a total weight of the core; and
b) a shell consisting of poly(lactic acid);
wherein in the system poly(lactic acid) is in a range from 40 wt.% to 50 wt.%, based on a total weight of the implantable system;
or
a) a core consisting of:
25 wt.% to 45 wt.% of 5-aminolevulinic acid and 75 wt.% to 55 wt.% of poly(lactic acid), based on a total weight of the core; and
b) a shell consisting of poly(lactic acid);
wherein in the system poly(lactic acid) is in a range from 75 wt.% to 85 wt.%, based on a total weight of the implantable system;
or
a) a core consisting of:
25 wt.% to 35 wt.% of 5-aminolevulinic acid and 75 wt.% to 65 wt.% of poly(ethylene oxide), based on a total weight of the core; and
b) a shell consisting of poly(lactic acid);
wherein in the system poly(lactic acid) is in a range from 45 wt.% to 55 wt.%, based on a total weight of the implantable system;
or
a) a core consisting of:
65 wt.% to 75 wt.% of 5-aminolevulinic acid and 35 wt.% to 25 wt.% of poly (lactic-co-glycolic acid), based on a total weight of the core; and
b) a shell consisting of 5 wt.% to 15 wt.% of 5-aminolevulinic acid, and 95 wt.% to 85 wt.% of poly(lactic acid) based on a total weight of the shell;
wherein in the system poly(lactic acid) is in a range from 35 wt.% to 45 wt.%, based on a total weight of the system;
or
a) a core consisting of:
25 wt.% to 30 wt.% of 5-aminolevulinic acid, 5 wt.% to 15 wt.% of citric acid or fumaric acid, and 60 wt.% to 70 wt.% of poly(lactic acid), based on a total weight of the core; and
b) a shell consisting of poly(lactic acid);
wherein in the system poly(lactic acid) is in a range from 75 wt.% to 85 wt.%, based on a total weight of the system;
or
a) a core consisting of:
65 wt.% to 75 wt.% of 5-aminolevulinic acid, and 35 wt.% to 25 wt.% of poly (lactic-co-glycolic acid) based on a total weight of the core; and
b) a shell consisting of 35 wt.% to 45 wt.% of poly(ethylene glycol) 400, and 55 wt.% to 65 wt.% of poly(lactic acid), based on a total weight of the shell
wherein in the system poly(lactic acid) is in a range from 25 wt.% to 35 wt.%, based on a total weight of the system.
or
a) a core consisting of:
25 wt.% to 45 wt.% of 5-aminolevulinic acid, and 75 wt.% to 55 wt.% of poly(lactic acid) or poly(ethylene glycol) or poly(ethylene oxide) based on the total weight of the core;
or
65 wt.% to 75 wt.% of 5-aminolevulinic acid, and 25 wt.% to 35 wt.% poly(lactic-co-glycolic acid) based on the total weight of the core;
and
b) a shell consisting of poly(lactic acid);
wherein in the system poly(lactic acid) is in a range from 20 wt.% to 60 wt.%, based on a total weight of the system.

7. The composition for use according to any one of claims 1 to 6, the composition further comprising an antioxidant selected from ascorbic acid, acetylcysteine, cysteine, thioglycerol, sodium hydrogen sulfite, butylated hydroxyanisole, butylated hydroxytoluene, α-tocopherol acetate, methionine, citric acid, ethylenediaminetetraacetic acid, tartaric acid, gallic acid and its esters, glutathione, uric acid, carotenoids, and polyphenols and/or an organic acid selected from citric acid, fumaric acid, glycolic acid, lactic acid, malic acid, tartaric acid, maleic acid, succinic acid and a mixture of two or more of these organic acids.

8. The composition for use according to any one of claims 1 to 7, wherein the direct intracranial drug delivery system is a pump, preferably an osmotic pump.

9. The composition for use according to any one of claims 1 to 8 further in combination with a therapeutically effective amount of an artemisinin compound selected from **1a**, **1b**, **1c**, **1d**, and **1e** or a pharmaceutically acceptable salt thereof 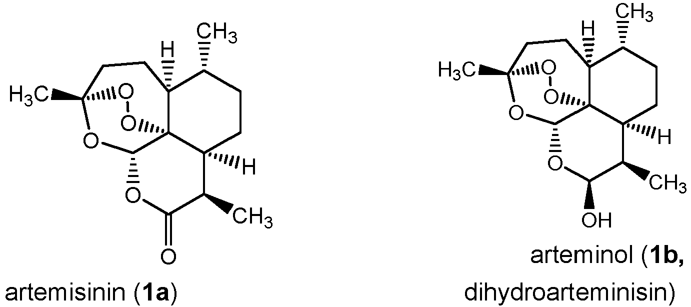 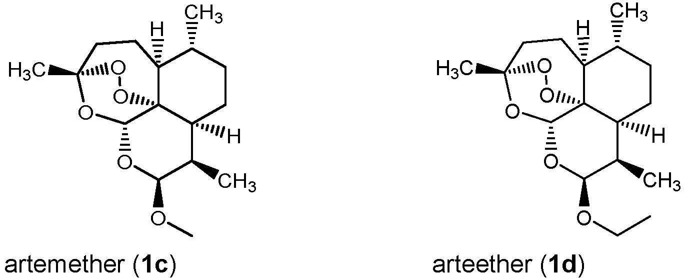 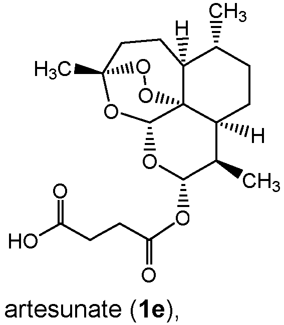 wherein the artemisinin compound is administered systemically or intracranially.

10. The composition for use according to claim 9, wherein the artemisinin compound is daily administered orally or intravenously to the subject during the release of the therapeutically effective amount of the composition into the resection cavity by the direct intracranial drug delivery system.

11. The composition for use according to claim 9 or 10, wherein the artemisinin compound is artesunate (**1e**).

12. The composition for use according to claim 10 or 11, wherein the daily dose of the artemisinin compound is in a range of 0.5 mg artemisinin compound per kg body weight to 10 mg artemisinin compound per kg body weight.

13. The composition for use according to any one of claims 1 to 12 further in combination with a therapeutically effective amount of at least one anti-glioblastoma drug selected from the group consisting of temozolomide, dexamethasone, lomustine, methotrexate, everolimus, carmustine, cyclophosphamide, cisplatin, carboplatin, 5-fluorouracil, triptolide, homoharringtonin, dactinomycin, doxorubicin, epirubicin, idarubicin, ribavirin, topotecan, flubendazole, itraconazole, vindesine sulfate, cerivastatin, vincristine, vinorebine, nisoldipine, deoxyadenosine, chloro-2'-deoxyadenosine, 5-nonyloxytryptamine, 2(1H)-pyrimidinone, pitavastatin, sertraline, irinotecan, clofazimine, tovorafenib, and docetaxel, wherein the therapeutically effective amount of at least one anti-glioblastoma drug is administered systemically daily to the subject during the release of the therapeutically effective amount of the composition into the resection cavity by the direct intracranial drug delivery system.

14. The composition for use according to claim 13, wherein the at least one anti-glioblastoma drug is selected from temozolomide, lomustine, cisplatin, and 5-fluorouracil.

15. The composition for use according to any one of claims 1 to 14 further in combination with a radiotherapy, photodynamic therapy, immunotherapy, electromagnetic field therapy, hyperthermia therapy, chemotherapy, cancer immunotherapy, cellular therapy such as Car-T and TIL.
